# EUROPEAN PATENT APPLICATION

(11) **EP 4 434 985 A1**
(43) Date of publication of application: **25.09.2024**
(21) Application number: 22894938.4
(22) Date of filing: 18.11.2022
(51) Int. Cl.: C07D 471/04, C07D 401/12, C07D 401/14, C07D 487/04, C07D 491/14, A61K 31/4375, A61K 31/498, A61P 35/00

(54) **SELECTIVE PARP1 INHIBITOR AND APPLICATION THEREOF**

(30) Priority: 19.11.2021 CN 202111375066; 14.12.2021 CN 202111522026; 14.01.2022 CN 202210026917; 30.09.2022 CN 202211196794
(71) Applicant: Kangbaida (Sichuan) Biotechnology Co., Ltd., Chengdu, Sichuan 610000 (CN)
(72) Inventor: ZHANG, Jing, Chengdu, Sichuan 610000 (CN); WEI, Yonggang, Chengdu, Sichuan 610000 (CN); SUN, Yi, Chengdu, Sichuan 610000 (CN)
(74) Representative: Kador & Partner Part mbB
(86) International application number: PCT/CN2022/132782
(87) International publication number: WO 2023/088408

(57) **Abstract**

A selective PARP1 inhibitor and an application thereof. Provided are a compound represented by general formula (I-A), and a stereoisomer, a pharmaceutically acceptable salt or a deuterated compound thereof. Also provided are a pharmaceutical composition comprising the compound or the stereoisomer thereof, and an application of the compound and the pharmaceutical composition in preparation of anti-tumour drugs.

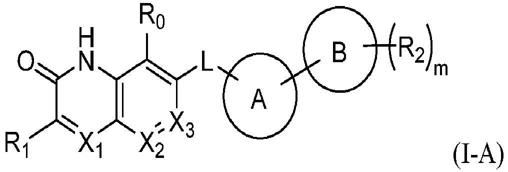

## Description

### TECHNICAL FIELD

The present invention relates to a selective PARP1 inhibitor or a stereoisomer thereof and the use thereof in medicine.

### BACKGROUND ART

PARPs (poly(ADP-ribose) polymerases) are a class of enzymes that catalyse the poly-ADP-ribosylation of various proteins. This process plays an important role in many cellular processes such as DNA damage repair, transcriptional regulation, and chromatin reorganization and remodelling. Currently, multiple PARP1/2 inhibitors are available on the market; however, whether used alone or in combination in clinical practice, they still exhibit common side effects on the blood and gastrointestinal tract, resulting in limited clinical application. Therefore, the development of safer and more effective PARP inhibitors remains an urgent clinical issue that needs to be addressed. A series of studies have shown that highly selective PARP1 inhibitors, when compared with PARP1/2 inhibitors, exhibit better efficacy and lower toxicity. Therefore, they are expected to mitigate the potential risks associated with the existing clinical PARP drugs, expand the range of clinical applications, and improve patients' quality of life.

### SUMMARY OF THE INVENTION

The present invention provides a selective PARP1 inhibitor or a stereoisomer thereof, and a pharmaceutical composition thereof, and the use thereof in medicine, wherein the compound described in the specification has high selectivity for and significant inhibitory activity on PARP1 and thus exhibits better efficacy and lower toxicity. In addition, the applicant has found in studies that these compounds can penetrate the blood-brain barrier. Therefore, the compound described herein or the stereoisomer thereof and the pharmaceutical composition thereof can also be used for the treatment of brain tumours.

One or more embodiments of the present invention provide a compound represented by general formula (I-A), or a stereoisomer, a pharmaceutically acceptable salt or a deuterated compound thereof: 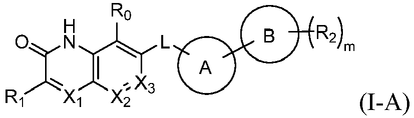 wherein:
R₁ is selected from C₁₋₆ alkyl, C₂₋₆ alkenyl, C₂₋₆ alkynyl, C₃₋₈ cycloalkyl or C₃₋₈ heterocycloalkyl, wherein the C₃₋₈ heterocycloalkyl may contain 1 to 4 heteroatoms selected from N, O or S;
R₀ is selected from H, halogen or C₁₋₆ alkyl, wherein the C₁₋₆ alkyl is optionally further substituted with one or more substituents selected from halogen;
X₁, X₂ and X₃ are each independently selected from N or CRx, and at least one of X₁, X₂ and X₃ is selected from N;
Rx is selected from H, halogen, hydroxyl, cyano, C₁₋₆ alkyl, C₁₋₆ alkoxy or C₃₋₈ cycloalkyl;
L is selected from CH₂;
A is a 4- to 12-membered heterocycle selected from a 4- to 12-membered monocyclic ring, a 5- to 12-membered spiro ring, a 4- to 12-membered fused ring, or a 4- to 12-membered bridged ring, wherein the 4- to 12-membered heterocycle may contain 1 to 4 heteroatoms selected from N, O or S;
structural fragment 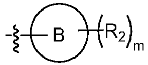 is selected from 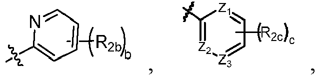 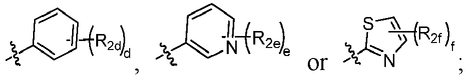
R_{2b} may be the same or different;
R_{2c} may be the same or different;
R_{2d} may be the same or different;
R₂ₑ may be the same or different;
R_{2f} may be the same or different;
R_{2b}, R_{2c} and R_{2f} are each independently selected from CN, halogen, OR₂ₐ, C₁₋₆ alkyl or a 4-to 12-membered heterocycle, wherein the C₁₋₆ alkyl and 4- to 12-membered heterocycle are optionally further substituted with one or more substituents selected from halogen, OH and C₁₋₃ alkyl, and the 4- to 12-membered heterocycle may contain 1 to 4 heteroatoms selected from N, O or S;
R_{2d} and R₂ₑ are each independently selected from CN, halogen, OR₂ₐ and C₁₋₆ alkyl, wherein the C₁₋₆ alkyl is optionally further substituted with one or more substituents selected from halogen or OH;
R₂ₐ is selected from H, C₁₋₆ alkyl, (CH₂)ₙC₃₋₈ cycloalkyl or (CH₂)ₙC₃₋₈ heterocycloalkyl, wherein the C₃₋₈ heterocycloalkyl may contain 1 to 4 heteroatoms selected from N, O or S, and the C₁₋₆ alkyl is optionally further substituted with one or more substituents selected from halogen;
Z₁, Z₂ and Z₃ are each independently selected from N or C, and at least two of Z₁, Z₂ and Z₃ are selected from N;
n is selected from 0, 1, 2 or 3;
b is selected from 1, 2 or 3;
c is selected from 1, 2 or 3;
d is selected from 2 or 3;
e is selected from 1, 2 or 3;
f is selected from 1 or 2,
provided that:
   the compound represented by general formula (I-A) is not:

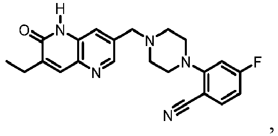

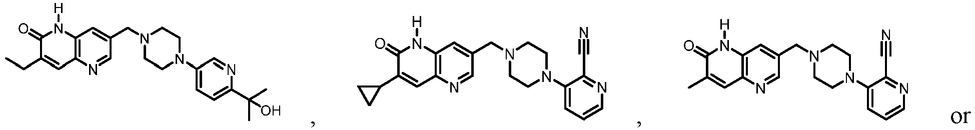

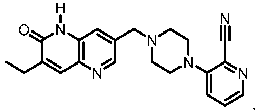

In a preferred embodiment, structure unit 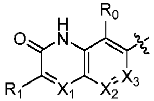 is selected from 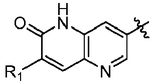 or 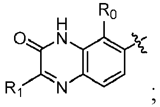
R₁ is selected from C₁₋₆ alkyl, C₂₋₆ alkenyl or C₃₋₈ cycloalkyl;
R₀ is selected from halogen;
A is selected from 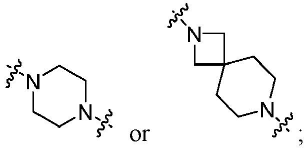
R_{2b} may be the same or different, and each R_{2b} is independently selected from CN, halogen, C₁₋₃ alkoxy, C₁₋₃ alkyl or a 4- to 12-membered heterocycle, wherein the C₁₋₃ alkyl, C₁₋₃ alkoxy and 4- to 12-membered heterocycle are optionally further substituted with one or more substituents selected from halogen, OH and C₁₋₃ alkyl;
R_{2c} is CN;
R_{2d} is CN or halogen;
R_{2f} is CN;
R₂ₑ may be the same or different, and each R₂ₑ is independently selected from CN, halogen, OR₂ₐ and C₁₋₆ alkyl, wherein the C₁₋₆ alkyl is optionally further substituted with one or more substituents selected from halogen or OH;
R₂ₐ is selected from C₁₋₃ alkyl or 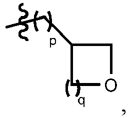 wherein the C₁₋₃ alkyl is optionally further substituted with one or more substituents selected from halogen;
p is selected from 0 or 1;
q is selected from 1 or 2.

In a further preferred embodiment, the structure unit 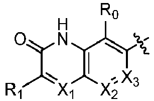 is selected from 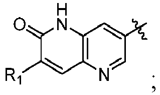
R₁ is selected from C₁₋₆ alkyl or C₃₋₈ cycloalkyl;
A is selected from 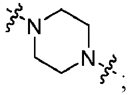
R₂ₑ may be the same or different, and each R₂ₑ is independently selected from CN, OR₂ₐ or C₁₋₃ alkyl, wherein the C₁₋₆ alkyl is optionally further substituted with one or more substituents selected from halogen;
R₂ₐ is selected from C₁₋₃ alkyl or 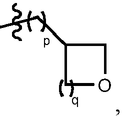 wherein the C₁₋₃ alkyl is optionally further substituted with one or more substituents selected from halogen.

In a further preferred embodiment, R_{2b} is selected from CN, halogen, C₁₋₃ alkyl or a 5-membered heterocycle, wherein the C₁₋₃ alkyl and 5-membered heterocycle are optionally further substituted with one or more substituents selected from halogen and C₁₋₃ alkyl;
R₂ₑ is selected from CN.

In a further preferred embodiment, R_{2b} is selected from CN.

One or more embodiments of the present invention provide a compound represented by general formula (I) or a stereoisomer thereof: 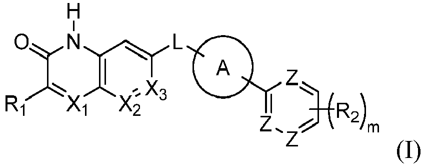 wherein:
R₁ is selected from C₁₋₆ alkyl, C₂₋₆ alkenyl, C₂₋₆ alkynyl, C₃₋₈ cycloalkyl or C₃₋₈ heterocycloalkyl, wherein the C₃₋₈ heterocycloalkyl may contain 1 to 4 heteroatoms selected from N, O or S;
X₁, X₂ and X₃ are each independently selected from N or CRx;
Rx is selected from H, halogen, hydroxyl, cyano, C₁₋₆ alkyl, C₁₋₆ alkoxy or C₃₋₈ cycloalkyl;
L is selected from CH₂;
A is a 4- to 12-membered heterocycle selected from a 4- to 12-membered monocyclic ring, a 5- to 12-membered spiro ring, a 4- to 12-membered fused ring, or a 4- to 12-membered bridged ring, wherein the 4- to 12-membered heterocycle may contain 1 to 4 heteroatoms selected from N, O or S;
R₂ may be the same or different, and each R₂ is independently selected from CN, halogen, OR₂ₐ or C₁₋₆ alkyl;
R₂ₐ is selected from H, C₁₋₆ alkyl, (CH₂)ₙC₃₋₈ cycloalkyl or (CH₂)ₙC₃₋₈ heterocycloalkyl, wherein the C₃₋₈ heterocycloalkyl may contain 1 to 4 heteroatoms selected from N, O or S, and the C₁₋₆ alkyl is optionally further substituted with one or more substituents selected from halogen;
Z may be the same or different, and each Z is independently selected from CH or N;
m is selected from 1, 2 or 3;
n is selected from 0, 1, 2 or 3,
provided that:
   the compound represented by general formula (I) is not

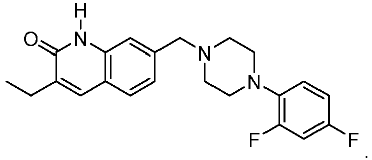

One or more embodiments of the present invention provide a compound represented by general formula (I) or a stereoisomer thereof: wherein: 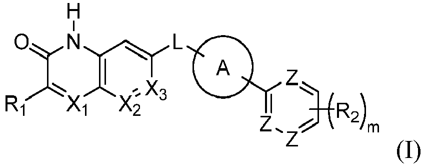
R₁ is selected from C₁₋₆ alkyl, C₂₋₆ alkenyl, C₂₋₆ alkynyl, C₃₋₈ cycloalkyl or C₃₋₈ heterocycloalkyl, wherein the C₃₋₈ heterocycloalkyl may contain 1 to 4 heteroatoms selected from N, O or S;
X₁, X₂ and X₃ are each independently selected from N or CRx;
Rx is selected from H, halogen, hydroxyl, cyano, C₁₋₆ alkyl, C₁₋₆ alkoxy or C₃₋₈ cycloalkyl;
L is selected from CH₂;
A is a 4- to 12-membered heterocycle selected from a 4- to 12-membered monocyclic ring, a 5- to 12-membered spiro ring, a 4- to 12-membered fused ring, or a 4- to 12-membered bridged ring, wherein the 4- to 12-membered heterocycle may contain 1 to 4 heteroatoms selected from N, O or S;
R₂ may be the same or different, and each R₂ is independently selected from CN, halogen, OR₂ₐ or C₁₋₆ alkyl, wherein the C₁₋₆ alkyl is optionally further substituted with one or more substituents selected from halogen or hydroxyl;
R₂ₐ is selected from H, C₁₋₆ alkyl, (CH₂)ₙC₃₋₈ cycloalkyl or (CH₂)ₙC₃₋₈ heterocycloalkyl, wherein the C₃₋₈ heterocycloalkyl may contain 1 to 4 heteroatoms selected from N, O or S, and the C₁₋₆ alkyl is optionally further substituted with one or more substituents selected from halogen;
Z may be the same or different, and each Z is independently selected from CH or N;
m is selected from 1, 2 or 3;
n is selected from 0, 1, 2 or 3,
provided that:
   the compound represented by general formula (I) is not

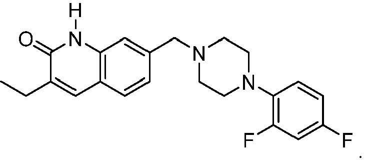

One or more embodiments of the present invention provide a compound represented by general formula (II) or a stereoisomer thereof: wherein: 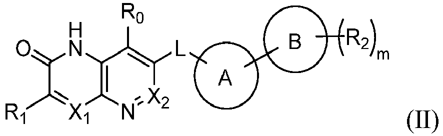
R₁ is selected from C₁₋₆ alkyl, C₂₋₆ alkenyl, C₂₋₆ alkynyl, C₃₋₈ cycloalkyl or C₃₋₈ heterocycloalkyl, wherein the C₃₋₈ heterocycloalkyl may contain 1 to 4 heteroatoms selected from N, O or S;
R₀ is selected from H, halogen or C₁₋₆ alkyl, wherein the C₁₋₆ alkyl is optionally further substituted with one or more substituents selected from halogen or C₁₋₆ alkyl;
X₁ and X₂ are each independently selected from Nor CRx;
Rx is selected from H, halogen, hydroxyl, cyano, C₁₋₆ alkyl, C₁₋₆ alkoxy or C₃₋₈ cycloalkyl;
L is selected from CH₂;
A is a 4- to 12-membered heterocycle selected from a 4- to 12-membered monocyclic ring, a 5- to 12-membered spiro ring, a 4- to 12-membered fused ring, or a 4- to 12-membered bridged ring, wherein the 4- to 12-membered heterocycle may contain 1 to 4 heteroatoms selected from N, O or S;
B is selected from 6-membered aryl or heteroaryl, wherein the heteroaryl may contain 1 to 4 heteroatoms selected from N;
R₂ may be the same or different, and each R₂ is independently selected from CN, halogen, OR₂ₐ or C₁₋₆ alkyl;
R₂ₐ is selected from H, C₁₋₆ alkyl, (CH₂)ₙC₃₋₈ cycloalkyl or (CH₂)ₙC₃₋₈ heterocycloalkyl, wherein the C₃₋₈ heterocycloalkyl may contain 1 to 4 heteroatoms selected from N, O or S, and the C₁₋₆ alkyl is optionally further substituted with one or more substituents selected from halogen;
Z may be the same or different, and each Z is independently selected from CH or N;
m is selected from 1, 2 or 3;
n is selected from 0, 1, 2 or 3.

In one or more embodiments of the present invention, the compound of the present invention is selected from: 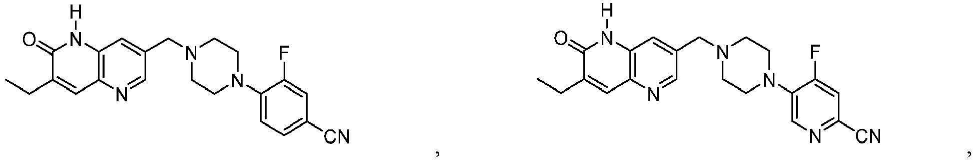 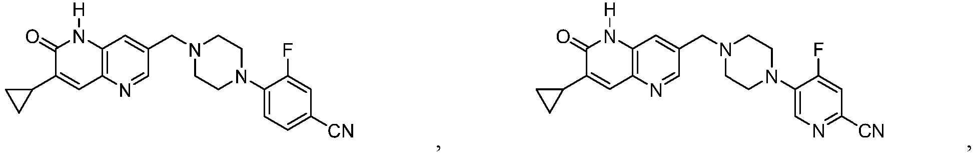 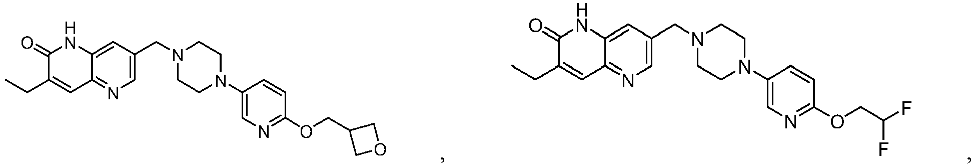 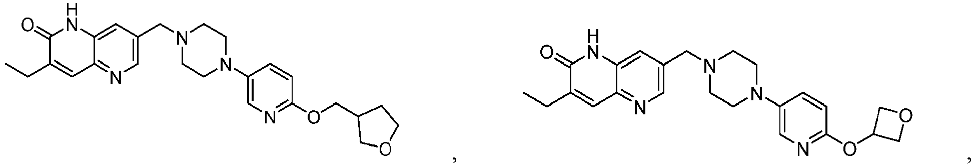 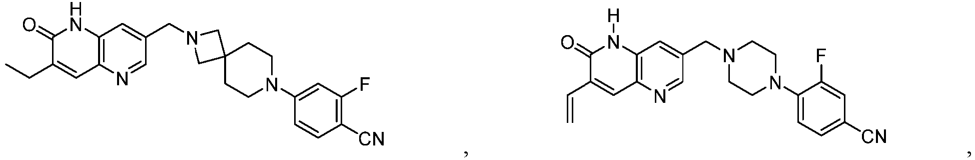 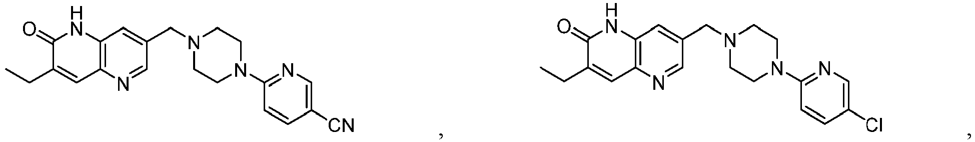 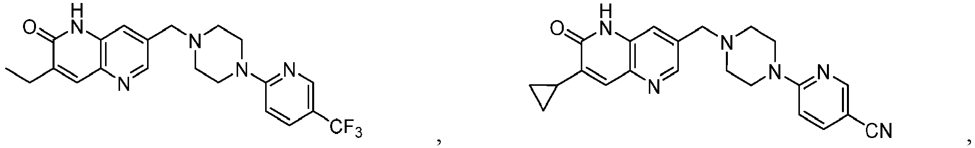 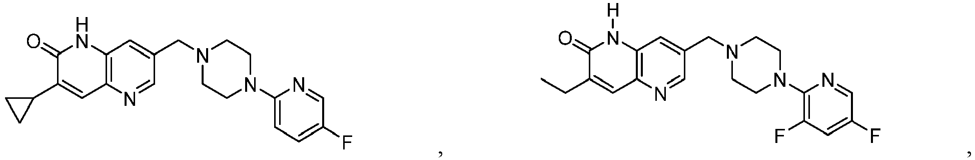 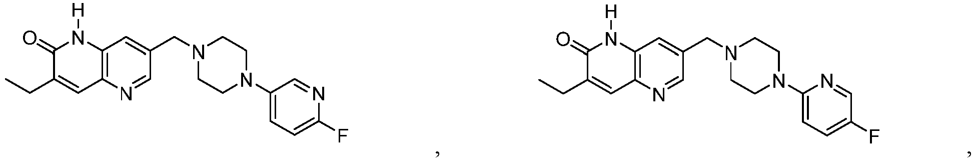 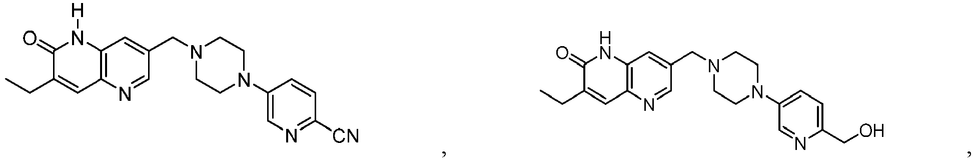 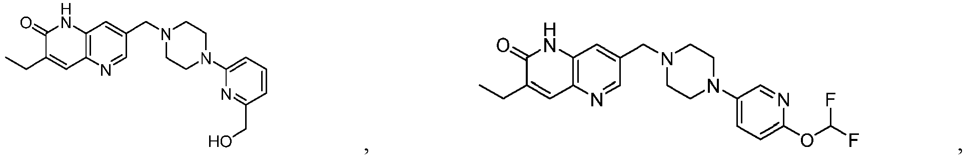 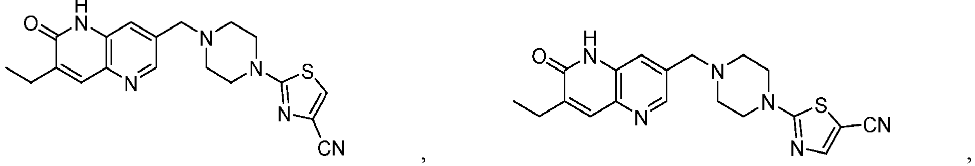 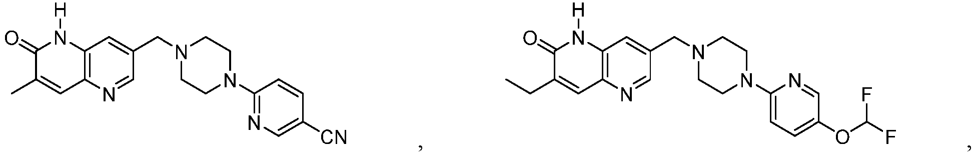 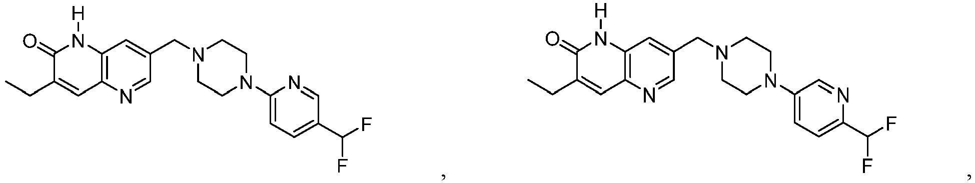 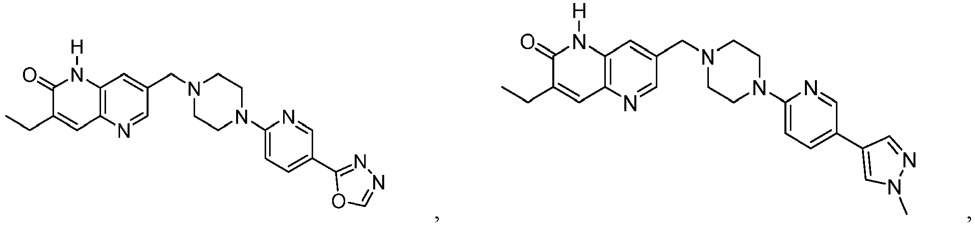 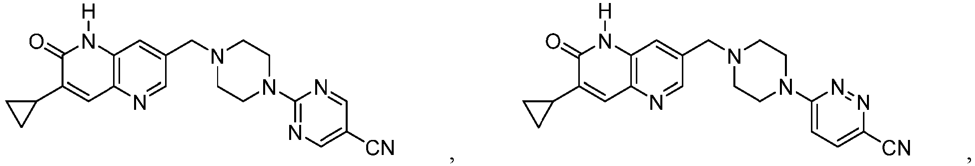 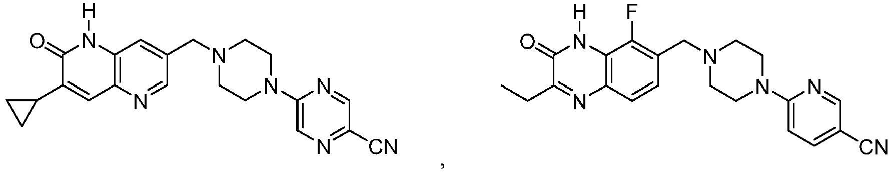 and

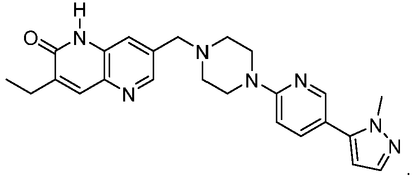

One or more embodiments of the present invention provide a pharmaceutical composition comprising:
(1) the compound or the stereoisomer thereof of the present invention;
(2) optionally one or more additional active ingredients; and
(3) a pharmaceutically acceptable carrier and/or excipient.

One or more embodiments of the present invention provide the use of the compound or the stereoisomer thereof of the present invention or the pharmaceutical composition of the present invention in the preparation of a drug for treating cancer.

In another aspect, one or more embodiments of the present invention relate to the compound or the stereoisomer thereof of the present invention or the pharmaceutical composition of the present invention for use in the treatment of cancer.

In another aspect, one or more embodiments of the present invention relate to a method for treating cancer, the method comprising administering a therapeutically effective amount of the compound or the stereoisomer thereof of the present invention or the pharmaceutical composition of the present invention.

Unless stated to the contrary, the terms used in the specification and claims have the following meanings.

Carbon, hydrogen, oxygen, sulphur, nitrogen, F, Cl, Br and I involved in the groups and compounds described herein are each inclusive of isotopes thereof, and the carbon, hydrogen, oxygen, sulphur or nitrogen involved in the groups and compounds of the present invention is optionally further replaced with one or more of their corresponding isotopes, wherein the isotopes of carbon include ¹²C, ¹³C and ¹⁴C, the isotopes of hydrogen include protium (H), deuterium (D, also known as heavy hydrogen), tritium (T, also known as superheavy hydrogen), the isotopes of oxygen include ¹⁶O, ¹⁷O and ¹⁸O, the isotopes of sulphur include ³²S, ³³S, ³⁴S and ³⁶S, the isotopes of nitrogen include ¹⁴N and ¹⁵N, the isotopes of fluorine include ¹⁷F and ¹⁹F, the isotopes of chlorine include ³⁵Cl and ³⁷Cl, and the isotopes of bromine include ⁷⁹Br and ⁸¹Br.

"Alkyl" refers to a linear or branched saturated aliphatic hydrocarbon group containing 1 to 20 carbon atoms, preferably alkyl containing 1 to 8 carbon atoms, more preferably alkyl containing 1 to 6 carbon atoms, further preferably alkyl containing 1 to 4 carbon atoms. Non-limiting examples include methyl, ethyl, n-propyl, isopropyl, n-butyl, sec-butyl, neobutyl, tert-butyl, n-pentyl, isopentyl, neopentyl, n-hexyl and various branched chain isomers thereof. When substituted, the alkyl may be optionally further substituted with one or more substituents.

"Cycloalkyl" refers to a saturated cyclic hydrocarbon group, the ring of which may be a 3- to 10-membered monocyclic ring, a 4- to 12-membered bicyclic ring or a 10- to 20-membered polycyclic ring system, and has preferably 3 to 10 ring carbon atoms, and more preferably 3 to 8 ring carbon atoms. Non-limiting examples of the "cycloalkyl" include cyclopropyl, cyclobutyl, cyclopentyl, cyclohexyl, cycloheptyl, cyclooctyl, cyclopropenyl, cyclobutenyl, cyclopentenyl, cyclohexenyl, cycloheptenyl, 1,5-cyclooctadienyl, 1,4-cyclohexadienyl and cycloheptatrienyl, etc. When substituted, the cycloalkyl may be optionally further substituted with zero or more substituents.

"Heterocycloalkyl" refers to a substituted or unsubstituted saturated non-aromatic cyclic group, which may be a 3- to 8-membered monocyclic ring, a 4- to 12-membered bicyclic ring or a 10- to 15-membered tricyclic ring system, contains 1 to 3 heteroatoms selected from N, O or S, and is preferably 3- to 8-membered heterocyclyl. The optionally substituted N and S in the ring of the "heterocycloalkyl" may be oxidized into various oxidation states; the "heterocycloalkyl" may be connected to a heteroatom or a carbon atom; and the "heterocycloalkyl" may be a bridged ring or a spiro ring. Non-limiting examples of the "heterocycloalkyl" include oxiranyl, aziridinyl, oxetanyl, azetidinyl, 1,3-dioxolanyl, 1,4-dioxolanyl, 1,3-dioxanyl, azepanyl, piperidinyl, piperidyl, morpholinyl, thiomorpholinyl, 1,3-dithianyl, tetrahydrofuryl, tetrahydropyrrolyl, tetrahydroimidazolyl, tetrahydrothiazolyl, tetrahydropyranyl, azabicyclo[3.2.1]octyl, azabicyclo[5.2.0]nonyl, oxatricyclo[5.3.1.1]dodecyl, azaadamantyl and oxaspiro[3.3]heptanyl.

"Alkenyl" refers to a linear or branched unsaturated aliphatic hydrocarbon group which contains 1 to 10 (e.g., 1, 2, 3, 4, 5, 6, 7, 8, 9 and 10) carbon-carbon double bonds and is composed of 2 to 20 carbon atoms, and preferably alkenyl containing 2 to 12 (e.g., 2, 3, 4, 5, 6, 7, 8, 9, 10, 11 and 12) carbon atoms, more preferably alkenyl containing 2 to 8 carbon atoms, further preferably alkenyl containing 2 to 6 carbon atoms. Non-limiting examples include vinyl, propen-2-yl, buten-2-yl, buten-2-yl, penten-2-yl, penten-4-yl, hexen-2-yl, hexen-3-yl, hepten-2-yl, hepten-3-yl, hepten-4-yl, octen-3-yl, nonen-3-yl, decen-4-yl and undecen-3-yl. The alkenyl may be optionally further substituted with one or more substituents.

"Alkynyl" refers to a linear or branched unsaturated aliphatic hydrocarbon group which contains 1 to 3 carbon-carbon triple bonds and is composed of 2 to 20 carbon atoms, and is preferably alkynyl containing 2 to 12 carbon atoms, more preferably alkynyl containing 2 to 8 carbon atoms, further preferably alkynyl containing 2 to 6 carbon atoms. Non-limiting examples include ethynyl, propyn-1-yl, propyn-2-yl, butyn-1-yl, butyn-2-yl, butyn-3-yl, 3,3-dimethylbutyn-2-yl, pentyn-1-yl, pentyn-2-yl, hexyn-1-yl, 1-heptyn-1-yl, heptyn-3-yl, heptyn-4-yl, octyn-3-yl, nonyn-3-yl, decyn-4-yl, undecyn-3-yl and dodecyn-4-yl. The alkynyl may be optionally further substituted with 0 to 4 substituents selected from F, Cl, Br, I, alkyl, alkoxy, linear alkenyl, linear alkynyl, amino, nitro, cyano, mercapto, amido, carbocyclyl or heterocyclyl.

"Heterocycle" or "heterocyclyl" refers to a saturated or unsaturated aromatic heterocycle or non-aromatic heterocycle. When the "heterocycle" or "heterocyclyl" is an aromatic heterocycle, its definition is the same as the definition of the "heteroaryl" above. When the "heterocycle" or "heterocyclyl" is a non-aromatic heterocycle, it can be a 3- to 10-membered (e.g., 3-, 4-, 5-, 6-, 7-, 8-, 9- or 10-membered) monocyclic ring, a 4- to 12-membered (e.g., 4-, 5-, 6-, 7-, 8-, 9-, 10-, 11- or 12-membered) bicyclic ring or a 10- to 15-membered (e.g., 10-, 11-, 12-, 13-, 14- or 15-membered) tricyclic ring system, contains 1 to 4 (e.g., 1, 2, 3 or 4) heteroatoms selected from N, O or S, and is preferably 3- to 8-membered heterocyclyl. The optionally substituted 1 to 4 (e.g., 1, 2, 3 or 4) N and S in the ring of the "heterocyclyl" or "heterocycle" can be oxidized to various oxidation states; the "heterocyclyl" or "heterocycle" can be connected to a heteroatom or a carbon atom; and the "heterocyclyl" or "heterocycle" may be a fused ring, a bridged ring or a spiro ring. The "heterocyclyl" or "heterocycle" may be optionally further substituted with one or more substituents.

"Aryl" refers to a substituted or unsubstituted aromatic ring, which may be a 5- to 8-membered (e.g., 5-, 6-, 7- or 8-membered) monocyclic ring, a 5- to 12-membered (e.g., 5-, 6-, 7-, 8-, 9-, 10-, 11- or 12-membered) bicyclic ring or a 10- to 15-membered (e.g., 10-, 11-, 12-, 13-, 14- or 15-membered) tricyclic ring system and may be a bridged ring or a spiro ring. Non-limiting examples include phenyl and naphthyl. The aryl may be optionally further substituted with one or more substituents.

"Heteroaryl" refers to a substituted or unsubstituted aromatic ring, which may be a 3- to 8-membered (e.g., 3-, 4-, 5-, 6-, 7- or 8-membered) monocyclic ring and contains 1 to 6 (e.g., 1, 2, 3, 4, 5 and 6) heteroatoms selected from N, O or S, and is preferably 5- to 8-membered heteroaryl. The heteroaryl can be connected to a heteroatom or a carbon atom, and the heteroaryl may be a bridged ring or a spiro ring. Non-limiting examples include pyridyl, furyl, thienyl, pyranyl, pyrrolyl, pyrimidinyl, pyrazinyl, pyridazinyl and imidazolyl. The heteroaryl is optionally further substituted with one or more substituents.

When substituted, the above-mentioned "alkyl", "alkenyl", "alkynyl", "heterocycle", "heterocyclyl", "cycloalkyl", "heterocycloalkyl", "aryl" or "heteroaryl" may be optionally further substituted with 0, 1, 2, 3, 4, 5, 6, 7, 8, 9 or 10 substituents selected from F, Cl, Br, I, hydroxyl, mercapto, nitro, cyano, amino, C₁₋₆ alkylamino, =O, C₁₋₆ alkyl, C₁₋₆ alkoxy, C₂₋₆ alkenyl, C₂₋₆ alkynyl, -NR^{q4}R^{q5}, =NR^{q6}, -C(=O)OC₁₋₆ alkyl, -OC(=O)C₁₋₆ alkyl, -C(=O)NR^{q4}R^{q5}, C₃₋₈ cycloalkyl, C₃₋₈ heterocycloalkyl, C₆₋₁₀ aryl, C₅₋₁₀ heteroaryl, -C(=O)OC₆₋₁₀ aryl, -OC(=O)C₆₋₁₀ aryl, -OC(=O)C₅₋₁₀ heteroaryl, -C(=O)OC₅₋₁₀ heteroaryl, -OC(=O)C₃₋₈ heterocycloalkyl, - C(=O)OC₃₋₈ heterocycloalkyl, -OC(=O)C₃₋₈ cycloalkyl, -C(=O)OC₃₋₈ cycloalkyl, -NHC(=O)C₃₋₈ heterocycloalkyl, -NHC(=O)C₆₋₁₀ aryl, -NHC(=O)C₅₋₁₀ heteroaryl, -NHC(=O)C₃₋₈ cycloalkyl, - NHC(=O)C₃₋₈ heterocycloalkyl, -NHC(=O)C₂₋₆ alkenyl or -NHC(=O)C₂₋₆ alkynyl, wherein the substituent C₁₋₆ alkyl, C₁₋₆ alkoxy, C₂₋₆ alkenyl, C₂₋₆ alkynyl, C₃₋₈ cycloalkyl, C₃₋₈ heterocycloalkyl, C₆₋₁₀ aryl, C₅₋₁₀ heteroaryl, -NHC(=O)C₆₋₁₀ aryl, -NHC(=O)C₅₋₁₀ heteroaryl, -NHC(=O)C₃₋₈ heterocycloalkyl or -NHC(=O)C₃₋₈ cycloalkyl is optionally further substituted with 1 to 3 substituents selected from OH, F, Cl, Br, I, C₁₋₆ alkyl, C₁₋₆ alkoxy, -NR^{q4}R^{q5} or =O; R^{q1} is selected from C₁₋₆ alkyl, C₁₋₆ alkoxy or C₆₋₁₀ aryl; R^{q2} and R^{q3} are selected from H or C₁₋₆ alkyl; and R^{q4} and R^{q5} are selected from H, C₁₋₆ alkyl, -NH(C=NR^{q1})NR^{q2}R^{q3}, -S(=O)₂NR^{q2}R^{q3}, -C(=O)R^{q1} or - C(=O)NR^{q2}R^{q3}, and the C₁₋₆ alkyl is optionally further substituted with one or more substituents selected from OH, F, Cl, Br, I, C₁₋₆ alkyl, C₁₋₆ alkoxy, C₆₋₁₀ aryl, C₅₋₁₀ heteroaryl, C₃₋₈ cycloalkyl or C₃₋₈ heterocycloalkyl; or R^{q4}, R^{q5} and the N atom form a 3- to 8-membered heterocycle, wherein the ring may contain one or more heteroatoms selected from N, O or S.

"Pharmaceutical composition" refers to a mixture of one or more compounds of the present invention or pharmaceutically acceptable salts or prodrugs thereof and other chemical components, wherein "other chemical components" refer to pharmaceutically acceptable carriers, excipients and/or one or more other therapeutic agents.

"Carrier" refers to a material that does not cause significant irritation to an organism and does not eliminate the biological activity and characteristics of a compound administered.

"Excipient" refers to an inert substance added to a pharmaceutical composition to facilitate the administration of a compound. Non-limiting examples include calcium carbonate, calcium phosphate, sugar, starch, cellulose derivatives (including microcrystalline cellulose), gelatine, vegetable oils, polyethylene glycols, diluents, granulating agents, lubricants, adhesives and disintegrants.

"Stereoisomer" refers to an isomer produced as a result of different spatial arrangement of atoms in molecules, including cis-trans isomers, enantiomers and conformational isomers.

"Optional" or "optionally" or "selective" or "selectively" means that the events or circumstance subsequently described may but not necessarily occur, and the description includes the case where the events or circumstance occur and the case where that do not occur. For example, "heterocyclyl optionally substituted with alkyl" means that the alkyl may but not necessarily exist, and the description includes the case where the heterocyclyl is substituted with alkyl and the case where the heterocyclyl is not substituted with alkyl.

### DETAILED DESCRIPTION OF EMBODIMENTS

The technical solutions of the present invention will be illustrated in detail by the following examples, but the scope of protection of the present invention includes but is not limited thereto.

The structures of the compounds are determined by nuclear magnetic resonance (NMR) or (and) mass spectrometry (MS). The NMR shift (δ) is given in the unit of 10⁻⁶ (ppm). NMR is determined with Bruker Avance III 400 and Bruker Avance 300 nuclear magnetic resonance instrument; the solvents for determination are deuterated dimethyl sulphoxide (DMSO-d₆), deuterated chloroform (CDCl₃) and deuterated methanol (CD₃OD); and the internal standard is tetramethylsilane (TMS).

MS is measured with Agilent 6120B (ESI) and Agilent 6120B (APCI);

Yantai Huanghai HSGF254 or Qingdao GF254 silica gel plate is used as a thin layer chromatography silica plate, and the silica gel plate for the thin layer chromatography (TLC) is of the specification of 0.15 mm-0.20 mm, and the specification when a product is separated and purified by thin layer chromatography is 0.4 mm-0.5 mm.

For the column chromatography, Yantai Huanghai silica gel (200-300 mesh silica gel) is generally used as a carrier.

### Example 1

### 4-(4-((7-ethyl-6-oxo-5,6-dihydro-1,5-naphthyridin-3-yl)methyl)piperazin-1-yl)-3-fluorobenzonitrile (compound 1)

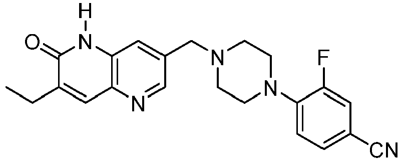

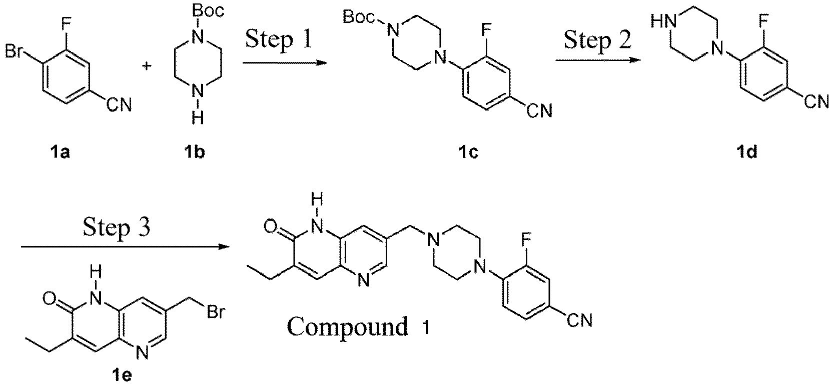

### Step 1

### Tert-butyl 4-(4-cyano-2-fluorophenyl)piperazine-1-carboxylate (1c)

4-bromo-3-fluorobenzonitrile (**1a**, 1 g, 5.00 mmol) and tert-butyl piperazine-1-carboxylate (**1b**, 838 mg, 4.5 mmol) were dissolved in toluene (15 mL), and then palladium acetate (112 mg, 0.5 mmol) and 2,2'-bis(diphenylphosphino)-1,1'-binaphthyl (311 mg, 0.5 mmol) were added. The reaction flask was subjected to nitrogen replacement and then reacted in an oil bath pan at 120°C for 16 h. The reaction mixture was quenched with water (20 mL) and extracted with ethyl acetate (3 × 30 mL). The organic phases were combined and concentrated under reduced pressure, and the crude product was purified by column chromatography (PE : EA = 5 : 1) to obtain compound 1c (yellow solid, 1.4 g, yield: 92%).

LCMS m/s = 306.15[M+1].

### Step 2:

### 3-fluoro-4-(piperazin-1-yl)benzonitrile (1d)

Hydrogen chloride 1,4-dioxane solution (4 M, 15 mL) was added to compound 1c (1.4 g, 4.60 mmol) and the mixture was stirred and reacted at room temperature for 16 h. The reaction liquid was filtered, and the filter cake was collected to obtain compound **1d** (yellow solid, 880 mg, yield: 94%).

¹H NMR (400 MHz, DMSO-*d₆*) δ 9.58(s, 1H), 7.69 (dd, J = 13.4, 2.0 Hz, 1H), 7.63-7.54 (m, 2H), 3.53-3.51 (m, 4H), 3.15-3.12 (m, 4H).

LCMS m/s = 205.10[M+1].

### Step 3:

### 4-(4-((7-ethyl-6-oxo-5,6-dihydro-1,5-naphthyridin-3-yl)methyl)piperazin-1-yl)-3-fluorobenzonitrile (compound 1)

Compound **1e** (white solid, 5 g, yield: 76%) was prepared according to the method for synthesizing **intermediate 14** in patent WO 2021013735, LCMS m/s = 267 [M+1].

Compound **1d** (100 mg, 0.49 mmol) and compound **1e** (130 mg, 0.49 mmol) were dissolved in acetonitrile (5 mL), and *N*,*N*-diisopropylethylamine (316 mg, 2.45 mmol) was added. The reaction system was subjected to nitrogen replacement and then reacted in an oil bath pan at 70°C for 3 h. The resulting solution was concentrated under reduced pressure to obtain a crude product, which was then purified by column chromatography (MeOH : DCM=1 : 60 to 1 : 15) to obtain **compound 1** (white solid, 139 mg, yield: 73%).

¹H NMR (400 MHz, DMSO-*d₆*) δ 11.86 (s, 1H), 8.40 (d, J = 1.8 Hz, 1H), 7.77-7.67 (m, 2H), 7.63-7.54 (m, 2H), 7.12 (t, J = 8.7 Hz, 1H), 3.64 (s, 2H), 3.21-3.17(m, 4H), 2.57-2.53 (m, 6H), 1.18 (t, J = 7.4 Hz, 3H).

LCMS m/s = 392.2[M+1].

### Example 2

### 7-((4-(3,5-difluoropyridin-2-yl)piperazin-1-yl)methyl)-3-ethyl-1,5-naphthyridin-2(1H)-one (compound 2)

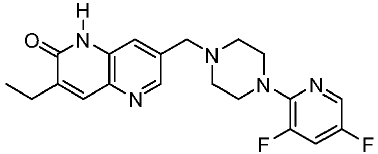

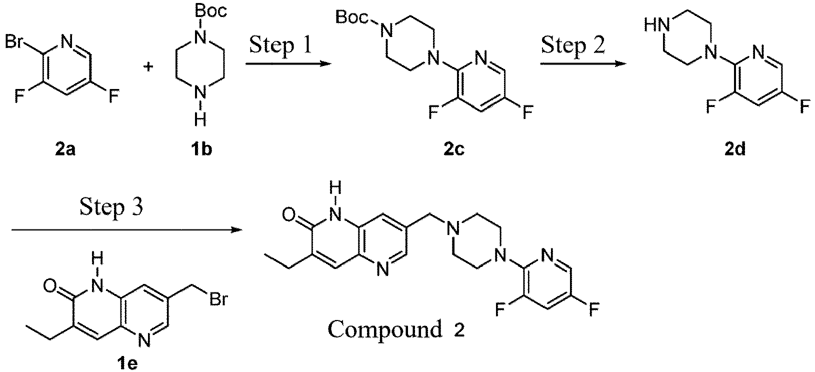

### Step 1

### Tert-butyl 4-(3,5-difluoropyridin-2-yl)piperazine-1-carboxylate (2c)

Intermediate **2c** (yellow solid, 1.8 g, yield: 85%) was prepared according to the method for synthesizing intermediate **1c.**

¹H NMR (400 MHz, DMSO-d6) δ 8.11 (d, 1H), 7.82 (ddd, 1H), 3.45 - 3.43 (m, 4H), 3.26 - 3.23 (m, 4H), 1.41 (s, 9H).

LCMS m/s = 300.10[M+1].

### Step 2

### 1-(3,5-difluoropyridin-2-yl)piperazine (2d)

Intermediate **2d** (yellow solid, 1.1 g, yield: 92%) was prepared according to the method for synthesizing intermediate **1d**.

¹H NMR (400 MHz, DMSO-d6) δ 9.46 (dr, 1H), 8.15 (d, 1H), 7.88 (ddd, 1H), 3.55 -3.52 (m, 4H), 3.20 - 3.16 (m, 4H).

LCMS m/s = 200.10[M+1].

### Step 3

### 7-((4-(3,5-difluoropyridin-2-yl)piperazin-1-yl)methyl)-3-ethyl-1,5-naphthyridin-2(1H)-one (compound 2)

**Compound 2** (white solid, 40 mg, yield: 40%) was prepared according to the method for synthesizing **compound 1.**

¹H NMR (600 MHz, DMSO-d6) δ 11.84 (s, 1H), 8.40 (d, 1H), 8.09 (d, 1H), 7.78 (ddd, 1H), 7.75 (s, 1H), 7.62 (d, 1H), 3.64 (s, 2H), 3.32 - 3.30 (m, 4H), 2.57 - 2.52 (m, 6H), 1.18 (t, 3H).

LCMS m/s = 386.20[M+1].

### Example 3

### 3-ethyl-7-((4-(5-fluoropyridin-2-yl)piperazin-1-yl)methyl)-1,5-naphthyridin-2(1H)-one (compound 3)

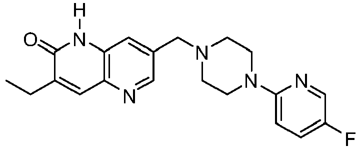

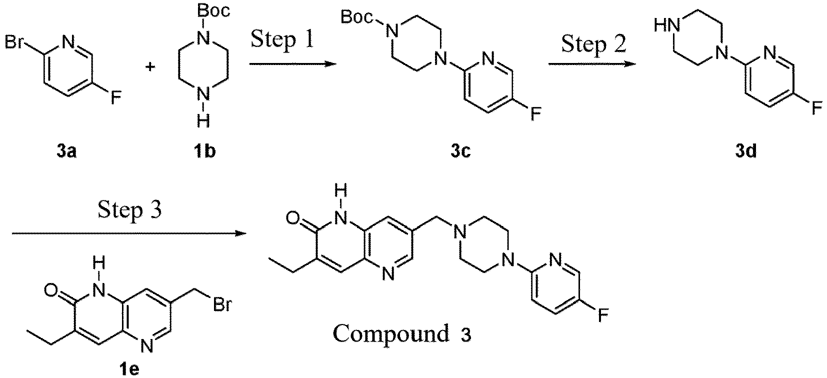

### Step 1

### tert-butyl 4-(5-fluoropyridin-2-yl)piperazine-1-carboxylate (3c)

Intermediate **3c** (yellow solid, 2 g, yield: 90%) was prepared according to the method for synthesizing intermediate **1c.**

¹H NMR (400 MHz, DMSO-d6) δ 8.10 (d, 1H), 7.53 (ddd, 1H), 6.89 (dd, 1H), 3.41 (s, 8H), 1.41 (s, 9H).

LCMS m/s = 282.20[M+1].

### Step 2

### 1-(5-fluoropyridin-2-yl)piperazine (3d)

Intermediate **3d** (yellow solid, 1.2 g, yield: 92%) was prepared according to the method for synthesizing intermediate **1d**.

¹H NMR (400 MHz, DMSO-d6) δ 9.55 (dr, 1H), 8.15 (d, 1H), 7.64 (td, 1H), 7.02 (dd, 1H), 3.72 - 3.69 (m, 4H), 3.21 - 3.01 (m, 4H).

LCMS m/s = 182.20[M+1].

### Step 3

### 3-ethyl-7-((4-(5-fluoropyridin-2-yl)piperazin-1-yl)methyl)-1,5-naphthyridin-2(1H)-one (compound 3)

**Compound 3** (white solid, 50 mg, yield: 45%) was prepared according to the method for synthesizing **compound 1.**

¹H NMR (400 MHz, DMSO-d6) δ 11.86 (s, 1H), 8.39 (d, 1H), 8.08 (d, 1H), 7.74 (s, 1H), 7.62 (d, 1H), 7.49 (ddd, 1H), 6.86 (dd, 1H), 3.62 (s, 2H), 3.48 - 3.38 (m, 4H), 2.53 (q, 2H), 2.50 - 2.46 (m, 4H), 1.17 (t, 3H).

LCMS m/s = 368.20[M+1].

### Example 4

### 5-(4-((7-ethyl-6-oxo-5,6-dihydro-1,5-naphthyridin-3-yl)methyl)piperazin-1-yl)picolinonitrile (compound 4)

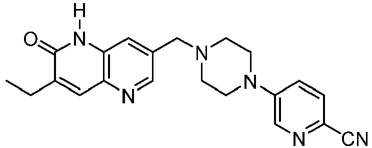

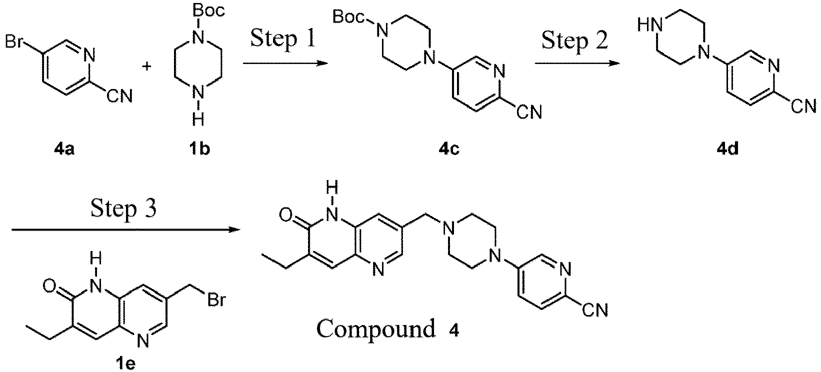

### Step 1

### Tert-butyl 4-(6-cyanopyridin-3-yl)piperazine-1-carboxylate (4c)

Intermediate **4c** (yellow solid, 1.3 g, yield: 88%) was prepared according to the method for synthesizing intermediate **1c.**

LCMS m/s = 289.16[M+1].

### Step 2

### 5-(piperazin-1-yl)picolinonitrile (4d)

Intermediate **4d** (yellow solid, 1.0 g, yield: 91%) was prepared according to the method for synthesizing intermediate **1d**.

LCMS m/s = 189.10[M+1].

### Step 3

### 5-(4-((7-ethyl-6-oxo-5,6-dihydro-1,5-naphthyridin-3-yl)methyl)piperazin-1-yl)picolinonitrile (compound 4)

**Compound 4** (white solid, 47 mg, yield: 51%) was prepared according to the method for synthesizing **compound 1.**

¹H NMR (400 MHz, DMSO-d6) δ 11.87 (s, 1H), 8.42 (d, J = 3.0 Hz, 1H), 8.40 (d, J = 1.9 Hz, 1H), 7.79 - 7.72 (m, 2H), 7.61 (s, 1H), 7.36 (dd, J = 8.9, 3.0 Hz, 1H), 3.64 (s, 2H), 3.43-3.40 (m, 4H), 2.58 - 2.51 (m, 6H), 1.18 (t, J = 7.4 Hz, 3H).

LCMS m/s = 375.16[M+1].

### Example 5

### 7-((4-(6-(2,2-difluoroethoxy)pyridin-3-yl)piperazin-1-yl)methyl)-3-ethyl-1, 5-naphthyridin-2(1H)-one (compound 5)

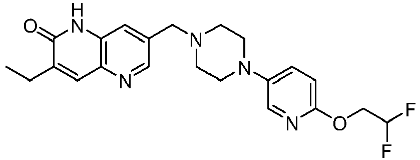

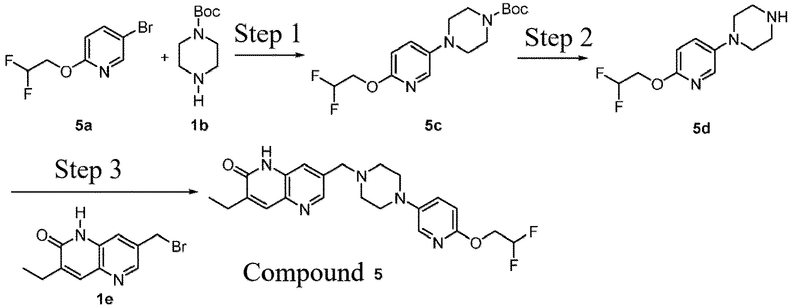

### Step 1

### Tert-butyl 4-(6-(2,2-difluoroethoxy)pyridin-3-yl)piperazine-1-carboxylate (5c)

Intermediate **5c** (yellow solid, 1.4 g, yield: 84%) was prepared according to the method for synthesizing intermediate **1c.**

¹H NMR (400 MHz, DMSO-d6) δ 7.80 (d, J = 3.0 Hz, 1H), 7.51 (dd, J = 9.0, 3.0 Hz, 1H), 6.83 (d, J = 9.0 Hz, 1H), 6.35 (tt, J = 54.9, 3.6 Hz, 1H), 4.47 (td, J = 15.0, 3.7 Hz, 2H), 3.45 (t, J = 5.1 Hz, 4H), 3.01 (t, J = 5.2 Hz, 4H), 1.41 (s, 9H).

LCMS m/s = 344.37[M+1].

### Step 2

### 1-(6-(2,2-difluoroethoxy)pyridin-3-yl)piperazine (5d)

Intermediate **5d** (yellow solid, 1.2 g, yield: 93%) was prepared according to the method for synthesizing intermediate **1d**.

¹H NMR (400 MHz, DMSO-d6) δ 9.46 (s, 1H), 7.85 (d, J = 3.0 Hz, 1H), 7.56 (dd, J = 9.0, 3.1 Hz, 1H), 7.09 (s, 1H), 6.51-6.22 (m, 1H), 4.53-4.46 (m, 2H), 3.31 (dd, J = 6.6, 3.7 Hz, 4H), 3.21-3.17 (m, 4H).

LCMS m/s = 244.26[M+1].

### Step 3

### 7-((4-(6-(2,2-difluoroethoxy)pyridin-3-yl)piperazin-1-yl)methyl)-3-ethyl-1, 5-naphthyridin-2(1H)-one (compound 5)

**Compound 5** (white solid, 63 mg, yield: 64%) was prepared according to the method for synthesizing **compound 1.**

¹H NMR (400 MHz, DMSO-d6) δ 11.86 (s, 1H), 8.39 (d, J = 1.8 Hz, 1H), 7.77 (d, J = 3.0 Hz, 1H), 7.75 (s, 1H), 7.62 (d, J = 1.8 Hz, 1H), 7.49 (dd, J = 9.0, 3.1 Hz, 1H), 6.80 (d, J = 9.0 Hz, 1H), 6.35 (tt, J = 55.0, 3.7 Hz, 1H), 4.46 (td, J = 15.0, 3.7 Hz, 2H), 3.64 (s, 2H), 3.08 (t, J = 4.7 Hz, 4H), 2.58-2.52 (m, 6H), 1.18 (t, J = 7.4 Hz, 3H).

LCMS m/s = 430.47M+1].

### Example 6

### 3-ethyl-7-((4-(6-((tetrahydrofuran-3-yl)methoxy)pyridin-3-yl)piperazin-1-yl)methyl)-1,5-naphthyridin-2(1H)-one (compound 6)

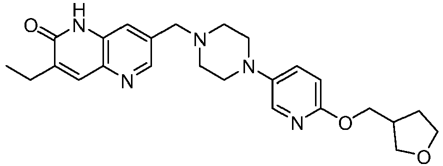

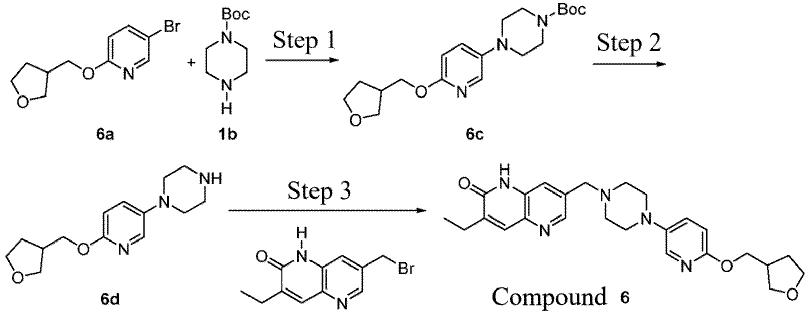

### Step 1

### Tert-butyl 4-(6-((tetrahydrofuran-3-yl)methoxy)pyridin-3-yl)piperazine-1-carboxylate (6c)

Intermediate **6c** (yellow solid, 1.1 g, yield: 87%) was prepared according to the method for synthesizing intermediate **1c.**

¹H NMR (400 MHz, DMSO-d6) δ 7.78 (d, J = 3.0 Hz, 1H), 7.46 (dd, J = 9.0, 3.0 Hz, 1H), 6.73 (d, J = 9.0 Hz, 1H), 4.68 (dd, J = 7.9, 6.0 Hz, 2H), 4.12-4.10 (m, 2H), 3.89 (dd, J = 6.5, 4.8 Hz, 2H), 3.45 (t, J = 5.0 Hz, 4H), 3.36 (dd, J = 7.9, 6.4 Hz, 1H), 2.98 (t, J = 5.1 Hz, 4H), 1.94-1.87 (m, 2H), 1.41 (s, 9H).

LCMS m/s = 364.46[M+1].

### Step 2

### 1-(6-((tetrahydrofuran-3-yl)methoxy)pyridin-3-yl)piperazine (6d)

Intermediate **6d** (yellow solid, 904 mg, yield: 90%) was prepared according to the method for synthesizing intermediate **1d**.

¹H NMR (400 MHz, DMSO-d6) δ 9.46 (s, 1H), 7.78 (d, J = 3.0 Hz, 1H), 7.46 (dd, J = 9.0, 3.0 Hz, 1H), 6.73 (d, J = 9.0 Hz, 1H), 4.68 (dd, J = 7.9, 6.0 Hz, 2H), 4.12-4.10 (m, 2H), 3.89 (dd, J = 6.5, 4.8 Hz, 2H), 3.45 (t, J = 5.0 Hz, 4H), 3.36 (dd, J = 7.9, 6.4 Hz, 1H), 2.98 (t, J = 5.1 Hz, 4H), 1.94-1.87 (m, 2H).

LCMS m/s = 264.34[M+1].

### Step 3

### 3-ethyl-7-((4-(6-((tetrahydrofuran-3-yl)methoxy)pyridin-3-yl)piperazin-1-yl)methyl)-1,5-naphthyridin-2(1H)-one (compound 6)

**Compound 6** (white solid, 41 mg, yield: 59%) was prepared according to the method for synthesizing **compound 1.**

¹H NMR (400 MHz, DMSO-d6) δ 11.94(s, 1H), 8.92 (s, 1H), 8.67(s, 1H), 7.87 (s, 1H), 7.84 (s, 1H), 7.47 (d, J = 8.9 Hz, 1H), 6.76 (d, J = 9.1 Hz, 1H), 4.54 (s, 1H), 4.25 - 4.00 (m, 2H), 3.79 - 3.71 (m, 2H), 3.67 - 3.56 (m, 6H), 3.44 (s, 1H), 3.24 (s, 1H), 3.06 (s, 2H), 2.67 - 2.51 (m, 4H), 1.99 (ddd, J = 15.3, 7.8, 4.1 Hz, 1H), 1.61 (dq, J = 13.3, 6.8 Hz, 1H), 1.19 (t, J = 7.4 Hz, 3H).

LCMS m/s = 450.56[M+1].

### Example 7

### 3-ethyl-7-((4-(6-(oxetan-3-ylmethoxy)pyridin-3-yl)piperazin-1-yl)methyl)-1,5-naphthyridin-2(1H)-one (compound 7)

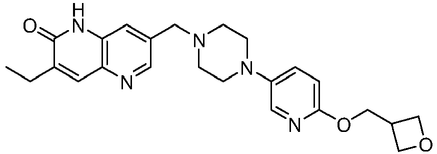

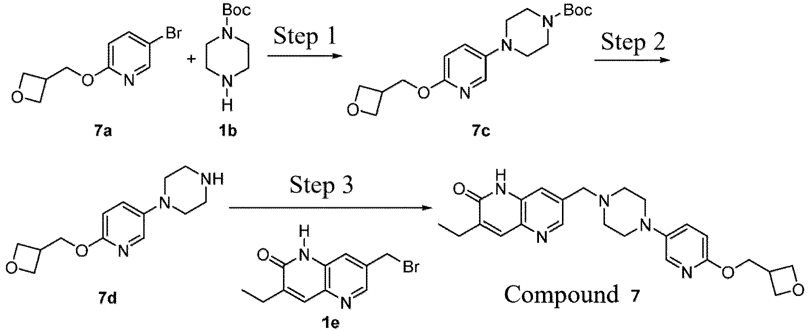

### Step 1

### Tert-butyl 4-(6-(oxetan-3-ylmethoxy)pyridin-3-yl)piperazine-1-carboxylate (7c)

Intermediate **7c** (yellow solid, 1.9 g, yield: 89%) was prepared according to the method for synthesizing intermediate **1c.**

¹H NMR (400 MHz, DMSO-d6) δ 7.78 (d, J = 3.0 Hz, 1H), 7.46 (dd, J = 9.0, 3.0 Hz, 1H), 6.73 (d, J = 9.0 Hz, 1H), 4.68 (dd, J = 7.9, 6.0 Hz, 2H), 4.39 (dd, J = 6.5, 4.8 Hz, 4H), 3.45 (t, J = 5.0 Hz, 4H), 3.36 (dd, J = 7.9, 6.4 Hz, 1H), 2.98 (t, J = 5.1 Hz, 4H), 1.41 (s, 9H).

LCMS m/s = 350.43[M+1].

### Step 2

### 1-(6-(oxetan-3-ylmethoxy)pyridin-3-yl)piperazine (7d)

Intermediate **7d** (yellow solid, 1.4 g, yield: 86%) was prepared according to the method for synthesizing intermediate **1d**.

LCMS m/s = 250.31[M+1].

### Step 3

### 3-ethyl-7-((4-(6-(oxetan-3-ylmethoxy)pyridin-3-yl)piperazin-1-yl)methyl)-1,5-naphthyridin-2(1H)-one (compound 7)

**Compound 7** (white solid, 37 mg, yield: 63%) was prepared according to the method for synthesizing **compound 1.**

¹H NMR (400 MHz, DMSO-d6) δ 11.94(s, 1H), 8.92 (s, 1H), 8.67(s, 1H), 7.87 (s, 1H), 7.84 (s, 1H), 7.47 (d, J = 8.9 Hz, 1H), 6.76 (d, J = 9.1 Hz, 1H), 4.54 (s, 1H), 4.39 (dd, J = 6.5, 4.8 Hz, 4H), 3.67-3.56 (m, 6H), 3.44 (s, 1H), 3.24 (s, 1H), 3.06 (s, 2H), 2.67-2.51 (m, 4H), 1.19 (t, J = 7.4 Hz, 3H).

LCMS m/s = 436.53[M+1].

### Example 8

### 3-ethyl-7-((4-(6-(hydroxymethyl)pyridin-2-yl)piperazin-1-yl)methyl)-1,5-naphthyridin-2(1H)-one (compound 8)

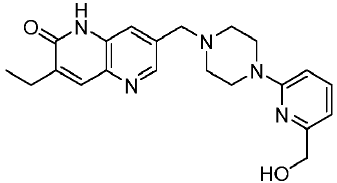

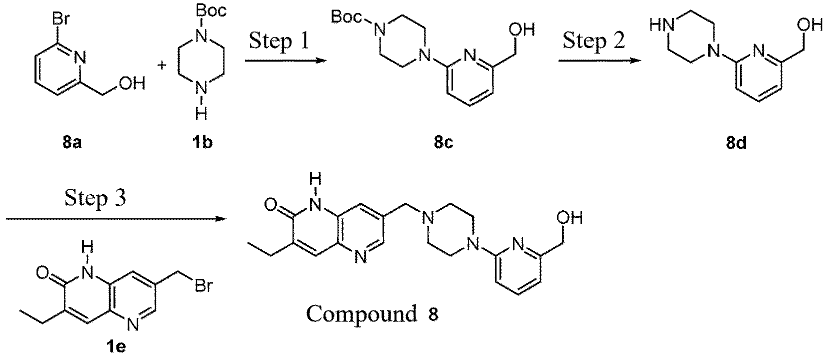

### Step 1

### Tert-butyl 4-(6-(hydroxymethyl)pyridin-2-yl)piperazine-1-carboxylate (8c)

Intermediate **8c** (yellow solid, 1.7 g, yield: 82%) was prepared according to the method for synthesizing intermediate **1c.**

¹H NMR (400 MHz, DMSO-*d₆*) δ 7.74 (d, 1H), 7.41 (dd, 1H), 6.95 (d, 1H), 5.33(t, 1H), 4.68 (dd, 2H), 3.45 (t, 4H), 2.98 (t, 4H), 1.41 (s, 9H).

LCMS m/s = 294.17[M+1].

### Step 2

### (6-(piperazin-1-yl)pyridin-2-yl)methanol (8d)

Intermediate **8d** (yellow solid, 1.2 g, yield: 87%) was prepared according to the method for synthesizing intermediate **1d**.

LCMS m/s = 194.12[M+1].

### Step 3

### 3-ethyl-7-((4-(6-(hydroxymethyl)pyridin-2-yl)piperazin-1-yl)methyl)-1,5-naphthyridin-2(1H)-one (compound 8)

**Compound 8** (white solid, 27 mg, yield: 55%) was prepared according to the method for synthesizing **compound 1.**

¹H NMR (400 MHz, DMSO-*d₆*) δ 11.64(s, 1H), 8.92 (s, 1H), 8.67(s, 1H), 7.87 (s, 1H), 7.84 (s, 1H), 7.47 (d, 1H), 7.09 (d, 1H), 5.64 (t, 1H),4.68 (dd, 2H), 3.45 (t, 4H), 3.06 (s, 2H), 2.98 (t, 4H), 2.43 (dd, 2H), 1.19 (t, 3H).

LCMS m/s = 436.53[M+1].

### Example 9

### 3-ethyl-7-((4-(5-(trifluoromethyl)pyridin-2-yl)piperazin-1-yl)methyl)-1,5-naphthyridin-2(1H)-one (compound 9)

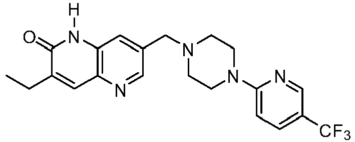

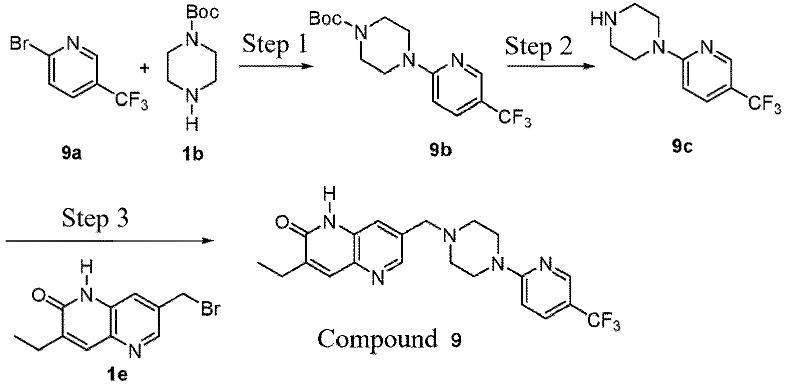

### Step 1

### Tert-butyl 4-(5-(trifluoromethyl)pyridin-2-yl)piperazine-1-carboxylate (9b)

Intermediate **9b** (yellow solid, 1.3 g, yield: 82%) was prepared according to the method for synthesizing intermediate **1c.**

LCMS m/s = 332.34[M+1].

### Step 2

### 1-(5-(trifluoromethyl)pyridin-2-yl)piperazine (9c)

Intermediate **9c** (yellow solid, 900 mg, yield: 89%) was prepared according to the method for synthesizing intermediate **1d**.

¹H NMR (400 MHz, DMSO-*d₆*) δ 9.73 (s, 1H), 8.45 (d, 1H), 7.90 (dd, 1H), 7.08 (d, 1H), 3.91 (t, 4H), 3.14 (p, 4H).

LCMS m/s = 232.22[M+1].

### Step 3

### 3-ethyl-7-((4-(5-(trifluoromethyl)pyridin-2-yl)piperazin-1-yl)methyl)-1,5-naphthyridin-2(1H)-one (compound 9)

**Compound 9** (white solid, 23 mg, yield: 71%) was prepared according to the method for synthesizing **compound 1.**

¹H NMR (400 MHz, DMSO-*d₆*) δ 11.87 (s, 1H), 8.40 (d, 2H), 7.79 (dd, 1H), 7.75 (d, 1H), 7.62 (d, 1H), 6.95 (d, 1H), 3.64 (d, 6H), 2.55 (td, 2H), 2.48 (t, 4H), 1.18 (t, 3H).

LCMS m/s = 418.44[M+1].

### Example 10

### 7-((4-(5-chloropyridin-2-yl)piperazin-1-yl)methyl)-3-ethyl-1,5-naphthyridin-2(1H)-one (compound 10)

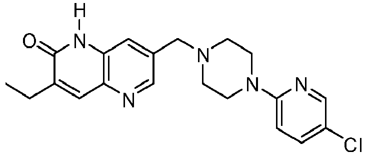

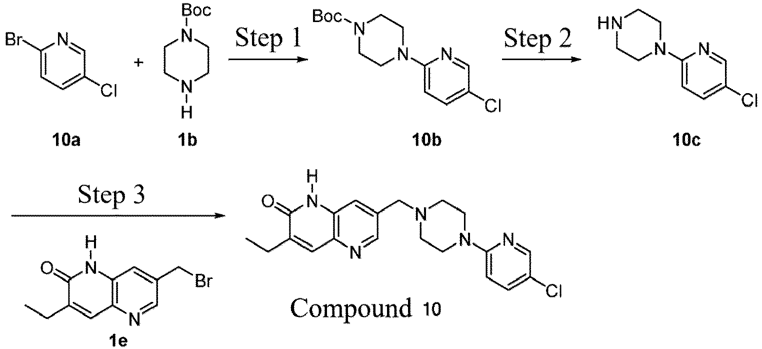

### Step 1

### Tert-butyl 4-(5-chloropyridin-2-yl)piperazine-1-carboxylate (10b)

Intermediate **10b** (yellow solid, 1.2 g, yield: 80%) was prepared according to the method for synthesizing intermediate 1c.

LCMS m/s = 298.78[M+1].

### Step 2

### 1-(5-chloropyridin-2-yl)piperazine (10c)

Intermediate **10c** (yellow solid, 930 mg, yield: 82%) was prepared according to the method for synthesizing intermediate **1d**.

LCMS m/s = 198.67[M+1].

### Step 3

### 7-((4-(5-chloropyridin-2-yl)piperazin-1-yl)methyl)-3-ethyl-1,5-naphthyridin-2(1H)-one (compound 10)

**Compound 10** (white solid, 46 mg, yield: 74%) was prepared according to the method for synthesizing **compound 1.**

¹HNMR(400 MHz, DMSO-*d₆*) δ 11.87 (s, 1H), 8.39 (d, 1H), 8.10 (d, 1H), 7.75 (d, 1H), 7.64 - 7.54 (m, 2H), 6.86 (d, 1H), 3.62 (s, 2H), 3.48 (t, 4H), 2.55 (td, 2H), 2.48 (t, 4H), 1.18 (t, 3H).

LCMS m/s = 384.88[M+1].

### Example 11

### 4-(4-((7-cyclopropyl-6-oxo-5,6-dihydro-1,5-naphthyridin-3-yl)methyl)piperazin-1-yl)-3-fluorobenzonitrile (compound 11)

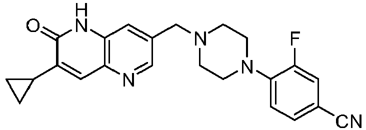

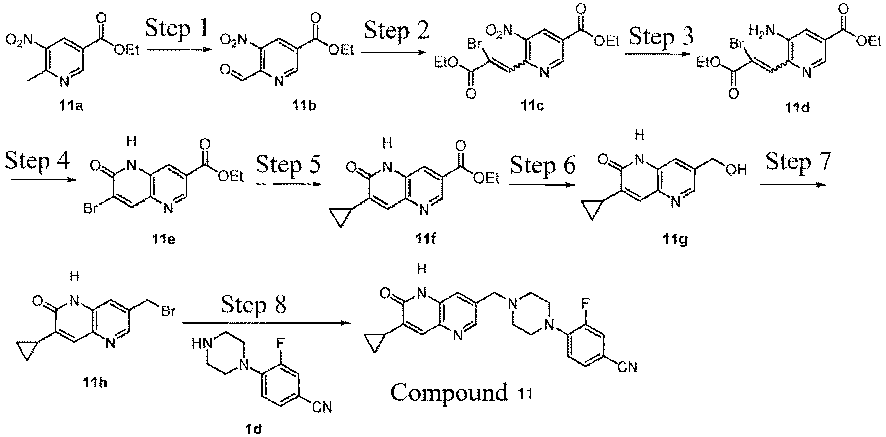

### Step 1

### Ethyl 6-formyl-5-nitronicotinate (11b)

Ethyl 6-methyl-5-nitronicotinate (**11a**, purchased from Jiangsu Aikang Biopharmaceutical R&D Co., Ltd., 10 g, 45.6 mmol) and selenium dioxide (7.6 g, 68.4 mmol) were dissolved in dioxane (100 mL). The mixture was refluxed at 110°C for 4 h. After the reaction was completed, the reaction mixture was subjected to hot filtration. The filtrate was concentrated under reduced pressure and subjected to column chromatography to obtain compound **11b** (yellow solid, 9.7 g, yield: 90%).

LC-MS m/z (ESI) = 225.10 [M+1].

### Step 2

### Ethyl 6-(2-bromo-3-ethoxy-3-oxoprop-1-en-1-yl)-5-nitronicotinate (11c)

Ethyl 2-bromo-2-(diethoxyphosphoryl)acetate (purchased from Meryer (Shanghai) Chemical Technology Co., Ltd., 20 g, 66.6 mmol) was dissolved in tetrahydrofuran (100 mL), and sodium hydride (1.6 g, 66.6 mmol) was added slowly at -78°C. The resulting mixture was heated slowly to 40°C, reacted for 10 min and then cooled to -78°C, and a solution of **11b** (9.7 g, 44.4 mmol) in tetrahydrofuran was added slowly dropwise. The mixture was reacted for 15 min, then quenched with saturated aqueous ammonium chloride solution (100 mL) and extracted with ethyl acetate (100 mL × 3). The organic phases were combined, concentrated under reduced pressure, and subjected to column chromatography to obtain **11c** (yellow solid, 13 g, yield: 81%, *E*/*Z* = 10 : 3).

¹H NMR (400 MHz, DMSO-*d*₆) δ 9.42 (d, 1H), 9.23 (d, 0.3H), 8.86 (d, 1H), 8.80 (d, 0.3H), 8.61 (s, 1H), 7.89 (s, 0.3H), 4.46-4.38 (m, 2.6H), 4.34 (q, 2H), 4.16 (q, 0.6H), 1.39-1.34 (m, 3.9H), 1.32 (t, 3H), 1.08 (t, 0.9H).

LC-MS m/z (ESI) = 373.00 [M+1].

### Step 3

### Ethyl 5-amino-6-(2-bromo-3-ethoxy-3-oxoprop-1-en-1-yl)nicotinate (11d)

Compound **11c** (13 g, 34.8 mmol) was dissolved in acetic acid (130 mL), and iron powder (5.8 g, 104.5 mmol) was added. The mixture was reacted at room temperature for 2 h, then quenched with distilled water (100 mL) and extracted with ethyl acetate (100 mL × 3). The organic phases were combined and concentrated under reduced pressure to obtain compound **11d** (yellow solid, 10 g, yield: 83%).

LC-MS m/z (ESI) = 343.00 [M+1].

### Step 4

Ethyl 7-bromo-6-oxo-5,6-dihydro-1,5-naphthyridine-3-carboxylate (**11e**)

Compound **11d** (10 g, 29.1 mmol) was placed in a reaction flask, and a solution of hydrogen bromide in acetic acid (100 mL) was added under nitrogen atmosphere. The mixture was reacted at 50°C for 4 h, and then concentrated under reduced pressure. Saturated aqueous sodium bicarbonate solution (100 mL) was added. The resulting solution was extracted with ethyl acetate (50 mL × 3), concentrated under reduced pressure and subjected to column chromatography to obtain compound **11e** (yellow solid, 2 g, yield: 23%).

¹H NMR (400 MHz, DMSO-*d*₆) δ 12.54 (s, 1H), 8.88 (d, 1H), 8.51 (s, 1H), 8.14 (d, 1H), 4.37 (q, 2H), 1.35 (t, 3H).

LC-MS m/z (ESI) = 297.00 [M+1].

### Step 5

### Ethyl 7-cyclopropyl-6-oxo-5,6-dihydro-1,5-naphthyridine-3-carboxylate (11f)

Compound **11e** (400 mg, 1.3 mmol), [1,1'-bis(diphenylphosphino)ferrocene]dichloropalladium-dichloromethane complex (purchased from Chengdu Dingdang Times Pharmaceutical Technology Co., Ltd., 328 mg, 0.40 mmol), potassium carbonate (745 mg, 5.4 mmol) and cyclopropylboronic acid (ACON Biotech (Hangzhou) Co., Ltd., 231 mg, 2.7 mmol) were dissolved in dioxane (4 mL). The resulting mixture was refluxed at 110°C for 8 h, quenched with water (5 mL), extracted with ethyl acetate (5 mL × 3), and concentrated under reduced pressure and the residue was purified by column chromatography to obtain compound **11f** (yellow solid, 270 mg, yield: 77%).

¹H NMR (400 MHz, DMSO-*d*₆) δ 12.07 (s, 1H), 8.85 (d, 1H), 8.12 (d, 1H), 7.46 (s, 1H), 4.36 (q, 2H), 2.25-2.12 (m, 1H), 1.34 (t, 3H), 1.02 (dt, 2H), 0.90 (dt, 2H).

LC-MS m/z (ESI) = 259.10 [M+1].

### Step 6

### 3-cyclopropyl-7-(hydroxymethyl)-1,5-naphthyridin-2(1H)-one (11g)

Compound **11f** (270 mg, 1 mmol) was dissolved in tetrahydrofuran (2 mL), and a solution of lithium aluminium hydride in tetrahydrofuran (purchased from Energy Chemical, 2 mL, 2 mmol) was added slowly dropwise in an ice-water bath. After the dropwise addition, the resulting solution was stirred for 10 min. Ethyl acetate (1 mL) was added, and the resulting mixture was concentrated under reduced pressure and separated by column chromatography to obtain compound **11g** (yellow solid, 100 mg, yield: 44%).

¹H NMR (400 MHz, DMSO-*d₆*) δ 11.92 (s, 1H), 8.35 (d, 1H), 7.59 (d, 1H), 7.41 (s, 1H), 5.45 (t, 1H), 4.60 (d, 2H), 2.16-2.09 (m, 1H), 0.96 (dt, 2H), 0.82 (dt, 2H).

LC-MS m/z (ESI) = 217.10 [M+1].

### Step 7

### 7-(bromomethyl)-3-cyclopropyl-1,5-naphthyridin-2(1H)-one (11h)

Compound **11g** (100 mg, 0.46 mmol) and triphenylphosphine (purchased from Shanghai Adamas Reagent Co., Ltd., 242 mg, 0.92 mmol) were dissolved in dichloromethane (1 mL). A solution of carbon tetrabromide (purchased from Energy Chemical, 306 mg, 0.92 mmol) in dichloromethane (0.5 mL) was added in an ice-water bath, and the mixture was reacted for 0.5 h. The reaction liquid was concentrated under reduced pressure and then subjected to column chromatography to obtain compound **11h** (yellow solid, 100 mg, yield: 78%).

LC-MS m/z (ESI) = 279.00 [M+1].

### Step 8

### 5-(4-((7-cyclopropyl-6-oxo-5,6-dihydro-1,5-naphthyridin-3-yl)methyl)piperazin-1-yl)-3-fluorobenzonitrile (compound 11)

Compound **11h** (100 mg, 0.36 mmol), compound **1d** (86 mg, 0.39 mmol) and *N*,*N-*diisopropylethylamine (230 mg, 1.8 mmol) were dissolved in acetonitrile (4 mL), and the resulting mixture was reacted at 80°C for 4 h. The reaction liquid was concentrated under reduced pressure and separated by Prep-HPLC to obtain **compound 11** (white solid, 40 mg, yield: 27%).

¹H NMR (400 MHz, DMSO-*d₆*) δ 11.89 (s, 1H), 8.37 (d, 1H), 7.69 (dd, 1H), 7.61 - 7.54 (m, 2H), 7.41 (s, 1H), 7.12 (t, 1H), 3.63 (s, 2H), 3.18 (t, 4H), 2.54 (t, 4H), 2.18 - 2.09 (m, 1H), 1.00 - 0.92 (m, 2H), 0.85 - 0.80 (m, 2H).

LC-MS m/z (ESI) = 404.46 [M+1].

### Example 12

### 3-cyclopropyl-7-((4-(5-fluoropyiidin-2-yl)piperazin-1-yl)methyl)-1,5-naphthyridin-2(1H)-one (compound 12)

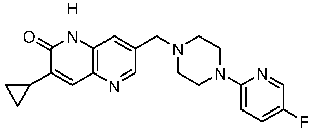

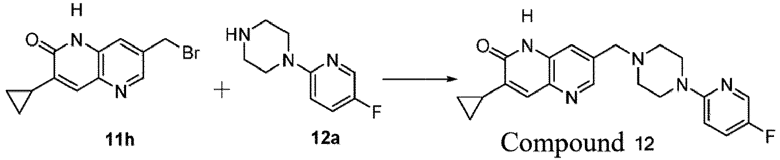

The method for synthesizing **compound 12** was the same as the method for synthesizing **compound 11.** The reaction liquid was concentrated under reduced pressure and separated by Prep-HPLC to obtain **compound 12** (white solid, 12 mg, yield: 24%).

¹H NMR (400 MHz, DMSO-*d₆*) δ 11.89 (s, 1H), 8.37 (d, 1H), 8.08 (d, 1H), 7.60 (d, 1H), 7.50 (td, 1H), 7.41 (s, 1H), 6.86 (dd, 1H), 3.61 (s, 2H), 3.42 (t, 4H), 2.49 - 2.43 (m, 4H), 2.14 (tt, 1H), 1.01 - 0.91 (m, 2H), 0.82 (dd, 2H).

LC-MS m/z (ESI) = 380.44 [M+1].

### Example 13

### 6-(4-((7-cyclopropyl-6-oxo-5,6-dihydro-1,5-naphthyridin-3-yl)methyl)piperazin-1-yl)nicotinonitrile (compound 13)

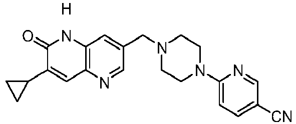

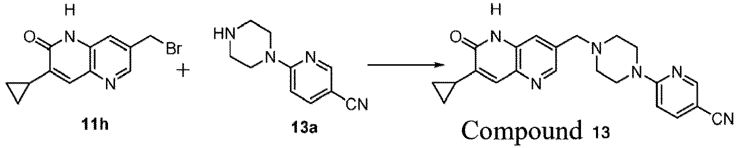

The method for synthesizing **compound 13** was the same as the method for synthesizing **compound 11.** The reaction liquid was concentrated under reduced pressure and separated by Prep-HPLC to obtain **compound 13** (white solid, 16 mg, yield: 22%).

¹H NMR (400 MHz, DMSO-*d₆*) δ 11.63 (s, 1H), 8.47 (d, 1H), 8.37 (d, 1H), 7.84 (dd, 1H), 7.59 (d, 1H), 7.41 (s, 1H), 6.92 (d, 1H), 3.66 (t, 4H), 3.61 (s, 2H), 2.46 (t, 4H), 2.13 (ddt, 1H), 1.00 - 0.91 (m, 2H), 0.86 - 0.76 (m, 2H).

LC-MS m/z (ESI) = 387.46 [M+1].

### Example 14

### 6-(4-((7-ethyl-6-oxo-5,6-dihydro-1,5-naphthyridin-3-yl)methyl)piperazin-1-yl)picolinonitrile (compound 14)

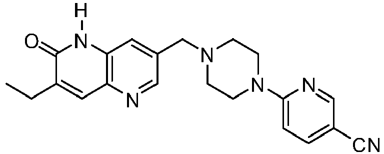

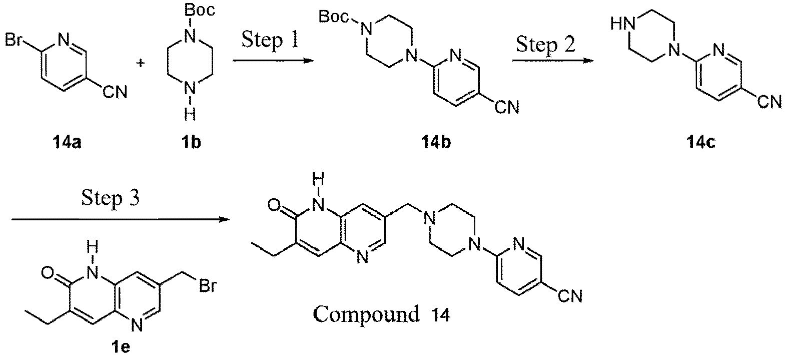

### Step 1

### Tert-butyl 4-(5-cyanopyridin-2-yl)piperazine-1-carboxylate (14b)

Intermediate **14b** (yellow solid, 1.2 g, yield: 85%) was prepared according to the method for synthesizing intermediate **1c.**

LCMS m/s = 289.16 [M+1].

### Step 2

### 6-(piperazin-1-yl)picolinonitrile (14c)

Intermediate **14c** (yellow solid, 1.1 g, yield: 90%) was prepared according to the method for synthesizing intermediate **1d**.

¹H NMR (400 MHz, DMSO-*d₆*) δ 9.59 (s, 1H), 8.54 (d, 1H), 7.95 (dd, 1H), 7.03 (d, 1H), 3.91 (t, 4H), 3.15 (t, 4H).

LCMS m/s = 189.10[M+1].

### Step 3

### 6-(4-((7-ethyl-6-oxo-5,6-dihydro-1,5-naphthyridin-3-yl)methyl)piperazin-1-yl)picolinonitrile (compound 14)

**Compound 14** (white solid, 43 mg, yield: 65%) was prepared according to the method for synthesizing **compound 1.**

¹H NMR (400 MHz, DMSO-*d₆*) δ 11.88 (s, 1H), 8.47 (d, 1H), 8.39 (d, 1H), 7.84 (dd, 1H), 7.74 (s, 1H), 7.62 (s, 1H), 6.93 (d, 1H), 3.67-3.65 (m, 4H), 3.63 (s, 2H), 2.58 - 2.52 (m, 2H), 2.48 - 2.44 (m, 4H), 1.17 (t, 3H).

LCMS m/s = 375.19 [M+1].

### Example 15

### 3-ethyl-7-((4-(5-(1-methyl-1H-pyrazol-5-yl)pyridin-2-yl)piperazin-1-yl)methyl)-1,5-naphthyridin-2(1H)-one (compound 15)

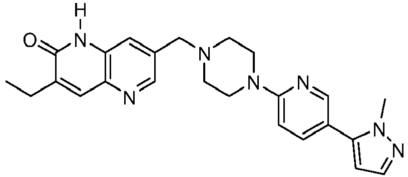

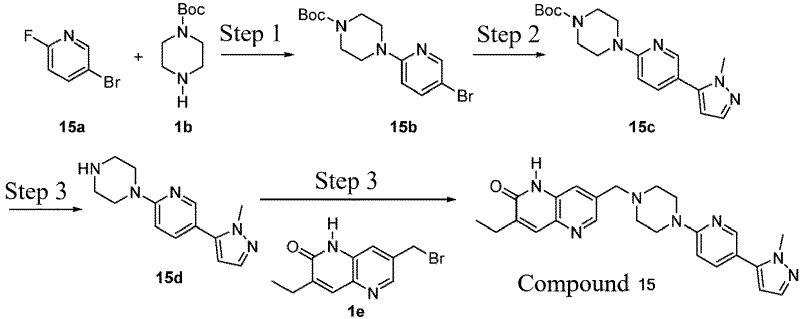

### Step 1

### Tert-butyl 4-(5-bromopyridin-2-yl)piperazine-1-carboxylate (15b)

5-bromo-2-fluoropyridine (**15a**, 1 g, 5.71 mmol) and tert-butyl piperazine-1-carboxylate (**1b**, 1.17 g, 4.5 mmol) were dissolved in dimethyl sulphoxide (20 mL), and potassium carbonate (1.18 g, 8.57 mmol) was added. The mixture in the reaction flask was reacted in an oil bath pan at 120°C for 8 h, and then the reaction was quenched with water (60 mL). A solid was precipitated out and filtered, and the filter cake was washed with water (3 mL*2) and dried to obtain compound **15b** (yellow solid, 1.7 g, yield: 87%).

LCMS m/s = 342.07 [M+1].

### Step 2

### Tert-butyl 4-(5-(1-methyl-1H-pyrazol-5-yl)pyridin-2-yl)piperazine-1-carboxylate (15c)

Tert-butyl 4-(5-bromopyridin-2-yl)piperazine-1-carboxylate (**15b**, 1.7 g, 5.00 mmol) and 1-methyl-1*H*-pyrazole-5-boronic acid pinacol ester (1.14 g, 5.50 mmol) were dissolved in 1,4-dioxane (20 mL). Methanesulphonato(2-dicyclohexylphosphino-2',4',6'-triisopropyl-1,1'-biphenyl)(2'-methylamino-1,1'-biphenyl-2-yl)palladium (II) (430.25 mg, 0.5 mmol) and caesium carbonate (3.26 g, 10.0 mmol) were added. The reaction flask was subjected to nitrogen replacement and then the mixture was reacted in an oil bath pan at 120°C for 16 h, quenched with water (50 mL) and extracted with ethyl acetate (3 × 30 mL). The organic phases were combined and concentrated under reduced pressure, and the crude product was purified by column chromatography (PE : EA = 3 : 1) to obtain compound **15c** (yellow solid, 1.5 g, yield: 88%).

LCMS m/s = 344.20[M+1].

### Step 3

### 1-(5-(1-methyl-1H-pyrazol-5-yl)pyridin-2-yl)piperazine (15d)

To compound **15c** (1.5 g, 4.37 mmol) was added hydrogen chloride 1,4-dioxane solution (4 M, 15 mL), and the mixture was stirred and reacted at room temperature for 2 h. The reaction liquid was filtered, and the filter cake was collected to obtain compound **15d** (yellow solid, 902.62 mg, yield: 85%).

LCMS m/s = 244.15 [M+1].

### Step 4

### 3-ethyl-7-((4-(5-(1-methyl-1H-pyrazol-5-yl)pyridin-2-yl)piperazin-1-yl)methyl)-1,5-naphthyridin-2(1H)-one (compound 15)

Compound **15d** (121.50 mg, 0.50 mmol) and compound **1e** (133.00 mg, 0.50 mmol) were dissolved in acetonitrile (5 mL), and *N*,*N*-diisopropylethylamine (316 mg, 2.50 mmol) was added. The reaction system was subjected to nitrogen replacement and then the mixture was reacted in an oil bath pan at 70°C for 3 h. The resulting solution was concentrated under reduced pressure to obtain a crude product, which was then purified by column chromatography (MeOH : DCM = 1 : 60 to 1 : 15) to obtain **compound 15** (white solid, 160.96 mg, yield: 75%).

¹H NMR (400 MHz, DMSO-*d₆*) δ 11.86 (s, 1H), 8.41 (d, 1H), 8.25 (d, 1H), 7.75 (s, 1H), 7.69 (dd, 1H), 7.63 (s, 1H), 7.43 (s, 1H), 6.92 (d, 1H), 6.34 (s, 1H), 3.81 (s, 3H), 3.64 (s, 2H), 3.59-3.52 (m, 4H), 3.36-3.33 (m, 4H), 2.56 (d, 2H), 1.18 (t, 3H).

LCMS m/s = 430.23 [M+1].

### Example 16

### 6-(4-((2-ethyl-5-fluoro-3-oxo-3,4-dihydroquinoxalin-6-yl)methyl)piperazin-1-yl)nicotinonitrile (compound 16)

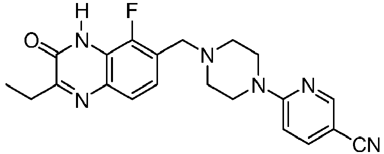

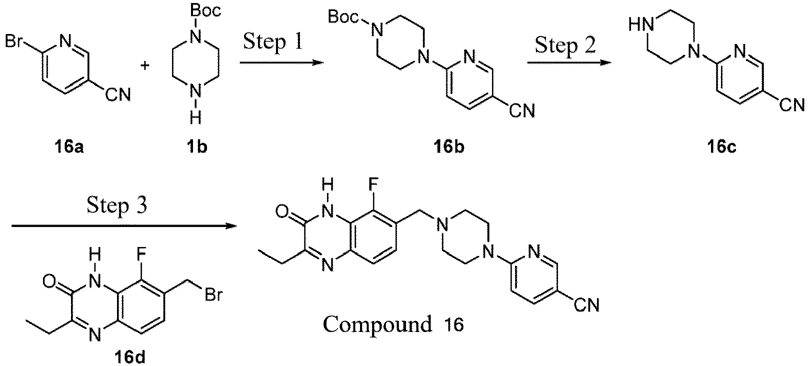

### Step 1

### Tert-butyl 4-(5-cyanopyridin-2-yl)piperazine-1-carboxylate (16b)

Intermediate **6b** (yellow solid, 1.2 g, yield: 85%) was prepared according to the method for synthesizing intermediate **1c.**

LCMS m/s = 289.16 [M+1].

### Step 2

### 6-(piperazin-1-yl)nicotinonitrile (16c)

Intermediate **16c** (yellow solid, 1.1 g, yield: 90%) was prepared according to the method for synthesizing intermediate **1d**.

¹H NMR (400 MHz, DMSO-*d₆*) δ 9.59 (s, 1H), 8.54 (d, 1H), 7.95 (dd, 1H), 7.03 (d, 1H), 3.91 (t, 4H), 3.15 (t, 4H).

LCMS m/s = 189.10[M+1].

### Step 3

### 6-(4-((2-ethyl-5-fluoro-3-oxo-3,4-dihydroquinoxalin-6-yl)methyl)piperazin-1-yl)nicotinonitrile (compound 16)

Compound **16d** (white solid, 5 g, yield: 76%) was prepared according to the method for synthesizing **intermediate 66** in patent WO 2021260092, LCMS m/s = 286.00 [M+1].

**Compound 16** (white solid, 52 mg, yield: 63%) was prepared according to the method for synthesizing **compound 1.**

¹H NMR (400 MHz, DMSO-*d*₆) δ 12.66 (s, 1H), 8.55 (s, 1H), 7.96 (d, 1H), 7.66 (d, 1H), 7.38 (t, 1H), 7.03 (d, 1H), 4.58-4.39 (m, 4H), 3.55 (s, 2H), 3.33 - 2.99 (m, 4H), 2.84 (q, 2H), 1.22 (t, 3H).

LCMS m/s = 394.17[M+1].

### Example 17

### 5-(4-((7-cyclopropyl-6-oxo-5,6-dihydro-1,5-naphthyridin-3-yl)methyl)piperazin-1-yl)pyrazine-2-carbonitrile (compound 17)

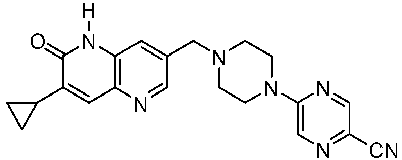

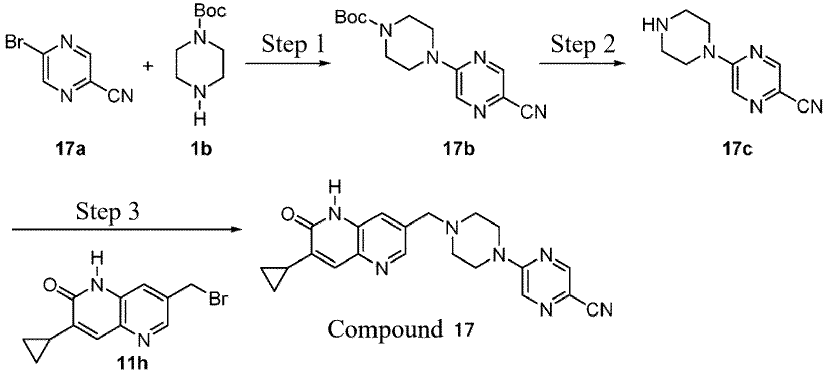

### Step 1

### Tert-butyl-4-(5-cyanopyrazin-2-yl)piperazine-1-carboxylate (17b)

Intermediate **17b** (yellow solid, 1.3 g, yield: 75%) was prepared according to the method for synthesizing intermediate **1c.**

LCMS m/s = 290.15 [M+1].

### Step 2

### 5-(piperazin-1-yl)pyrazine-2-carbonitrile (17c)

Intermediate **17c** (yellow solid, 0.9 g, yield: 90%) was prepared according to the method for synthesizing intermediate **1d**.

LCMS m/s = 190.10[M+1].

### Step 3

### 5-(4-((7-cyclopropyl-6-oxo-5,6-dihydro-1,5-naphthyridin-3-yl)methyl)piperazin-1-yl)pyrazine-2-carbonitrile (compound 17)

**Compound 17** (white solid, 23 mg, yield: 61%) was prepared according to the method for synthesizing **compound 1.**

¹H NMR (400 MHz, DMSO) *δ* 11.89 (s, 1H), 8.75 (s, 2H), 8.37 (d, 1H), 7.59 (d, 1H), 7.41 (s, 1H), 3.84 (t, 4H), 3.62 (s, 2H), 2.46 (t, 4H), 1.22 (dt, 1H), 1.01 - 0.92 (m, 2H), 0.82 (dt, 2H).

LCMS m/s = 388.19 [M+1].

### Example 18

### 6-(4-((7-cyclopropyl-6-oxo-5,6-dihydro-1,5-naphthyridin-3-yl)methyl)piperazin-1-yl)pyridazine-3-carbonitrile (compound 18)

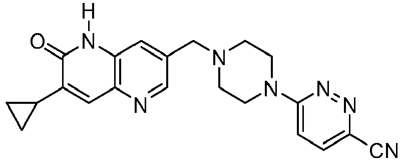

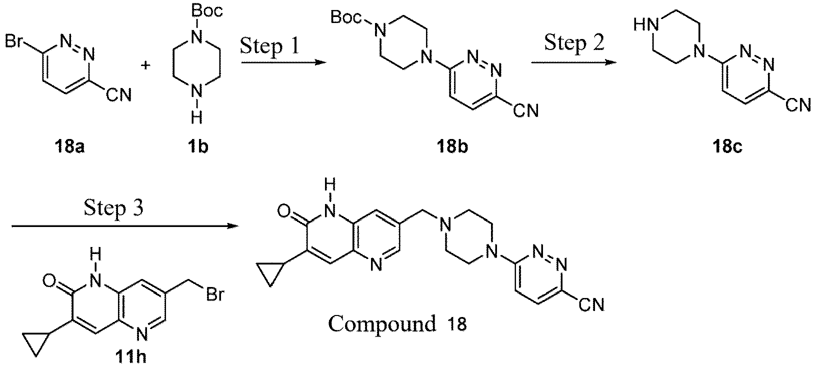

### Step 1

### Tert-butyl-4-(6-cyanopyridazin-3-yl)piperazine-1-carboxylate (18b)

Intermediate **18b** (yellow solid, 1.5 g, yield: 80%) was prepared according to the method for synthesizing intermediate **1c.**

LCMS m/s = 290.15 [M+1].

### Step 2

### 6-(piperazin-1-yl)pyridazine-3-carbonitrile (18c)

Intermediate **18c** (yellow solid, 1.1 g, yield: 88%) was prepared according to the method for synthesizing intermediate **1d**.

LCMS m/s = 190.10[M+1].

### Step 3

### 6-(4-((7-cyclopropyl-6-oxo-5,6-dihydro-1,5-naphthyridin-3-yl)methyl)piperazin-1-yl)pyridazine-3-carbonitrile (compound 18)

**Compound 18** (white solid, 23 mg, yield: 61%) was prepared according to the method for synthesizing **compound 1.**

¹H NMR (400 MHz, DMSO) δ 11.89 (s, 1H), 8.55 (d, 1H), 8.39 (d, 1H), 7.77 - 7.74 (m, 1H), 7.70 (dd, 1H), 7.65 (d, 1H), 3.64 (s, 2H), 3.53 (dd, 4H), 2.54 (d, 4H), 1.34 (t, 1H), 1.17 - 1.08 (m, 2H), 0.91 - 0.81 (m, 2H).

LCMS m/s = 388.19 [M+1].

### Example 19

### 2-(4-((7-cyclopropyl-6-oxo-5,6-dihydro-1,5-naphthyridin-3-yl)methyl)piperazin-1-yl)pyrimidine-5-carbonitrile (compound 19)

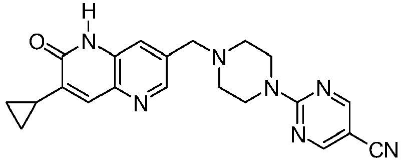

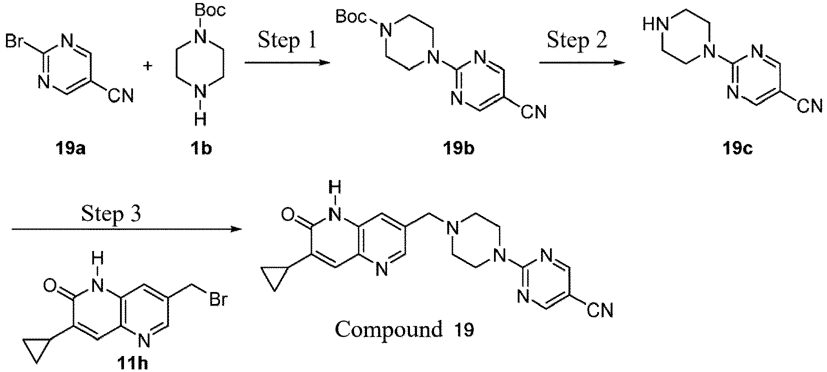

### Step 1

### Tert-butyl-4-(5-cyanopyrimidin-2-yl)piperazine-1-carboxylate (19b)

Intermediate **19b** (yellow solid, 1.1 g, yield: 74%) was prepared according to the method for synthesizing intermediate **1c.**

LCMS m/s = 290.15 [M+1].

### Step 2

### 2-(piperazin-1-yl)pyrimidine-5-carbonitrile (19c)

Intermediate **19c** (yellow solid, 0.8 g, yield: 89%) was prepared according to the method for synthesizing intermediate **1d**.

LCMS m/s = 190.10[M+1].

### Step 3

### 2-(4-((7-cyclopropyl-6-oxo-5,6-dihydro-1,5-naphthyridin-3-yl)methyl)piperazin-1-yl)pyrimidine-5-carbonitrile (compound 19)

**Compound 19** (white solid, 23 mg, yield: 61%) was prepared according to the method for synthesizing **compound 1.**

¹H NMR (400 MHz, DMSO) *δ* 11.89 (s, 1H), 8.78 (s, 2H), 8.34 (d, 1H), 7.57 (d, 1H), 7.43 (s, 1H), 3.93 - 3.81 (m, 4H), 3.61 (s, 2H), 2.43 - 2.28 (m, 4H), 1.21 (dt, 1H), 1.01 - 0.94 (m, 2H), 0.83 - 0.79 (m, 2H).

LCMS m/s = 388.19 [M+1].

### Example 21

### 3-ethyl-7-((4-(5-(1-methyl-1H-pyrazol-4-yl)pyridin-2-yl)piperazin-l-yl)methyl)-1,5-naphthyridin-2(1H)-one (compound 21)

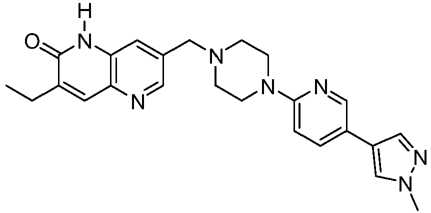

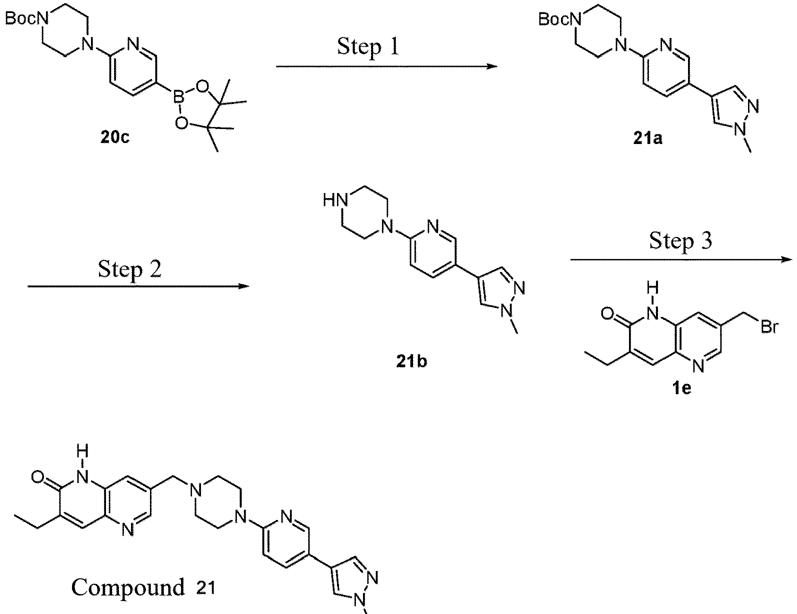

### Step 1

### Tert-butyl-4-(5-(1-methyl-1H-pyrazol-4-yl)pyridin-2-yl)piperazine-1-carboxylate (21a)

Intermediate **21a** (yellow solid, 330 mg, yield: 64%) was prepared according to the method for synthesizing intermediate **20d.**

LCMS m/s = 345.20[M+1].

### Step 2

### 1-(5-(1-methyl-1H-pyrazol-4-yl)pyridin-2-yl)piperazine (21b)

Intermediate **21b** (yellow solid, 120 mg, yield: 61%) was prepared according to the method for synthesizing intermediate **1d**.

LCMS m/s = 244.15[M+1].

### Step 3

### 3-ethyl-7-((4-(5-(1-methyl-1H-pyrazol-4-yl)pyridin-2-yl)piperazin-1-yl)methyl)-1,5-naphthyridin-2(1H)-one (compound 21)

**Compound 21** (white solid, 30 mg, yield: 71%) was prepared according to the method for synthesizing **compound 1.**

¹H NMR (400 MHz, DMSO) *δ* 11.86 (s, 1H), 8.41 (s, 1H), 8.34 (s, 1H), 8.02 (s, 1H), 7.74 - 7.69 (m, 3H), 7.64 (s, 1H), 6.84 (d, 1H), 3.84 (s, 3H), 3.64 - 3.47 (m, 6H), 2.68 - 2.62 (m, 4H),1.99 (t, 2H), 1.23 (s, 3H).

LCMS m/s = 429.24 [M+1]

### Example 22

### 7-((4-(5-(1,3,4-oxadiazol-2-yl)pyridin-2-yl)piperazin-1-yl)methyl)-3-ethyl-1,5-naphthyridin-2(1H)-one (compound 22)

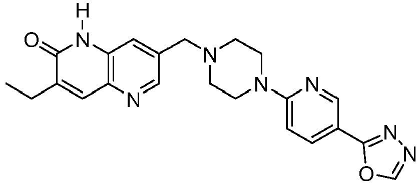

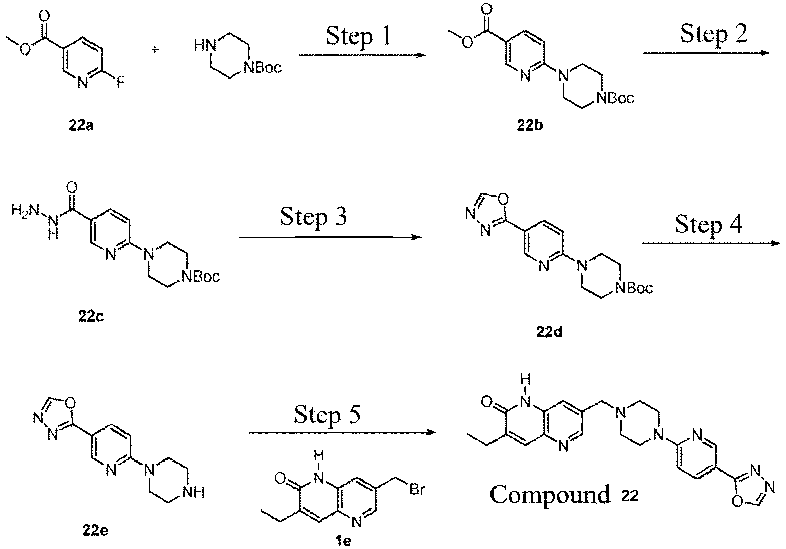

### Step 1

### Tert-butyl 4-(5-(methoxycarbonyl)pyridin-2-yl)piperazine-1-carboxylate (22b)

Intermediate **22b** (white solid, 8.4 g, yield: 76%) was prepared according to the method for synthesizing intermediate **4c.**

LCMS m/s = 322.17[M+1].

### Step 2

### Tert-butyl 4-(5-(hydrazinecarbonyl)pyridin-2-yl)piperazine-1-carboxylate (22c)

Intermediate **22b** (2.0 g, 6.2 mmol) was added to hydrazine hydrate (3.2 g, 62 mmol), and then the mixture was reacted in an oil bath at 80°C for 24 h. After the reaction was completed, the resulting mixture was concentrated under reduced pressure and the residue was dissolved with 50 ml of ethyl acetate. The organic phases were washed with water, dried over Na₂SO₄, and evaporated under reduced pressure to remove the solvent, to obtain crude **22c** (yellow solid, 820 mg, yield: 83%).

LCMS m/s = 322.18[M+1].

### Step 3

### Tert-butyl 4-(5-(1,3,4-oxadiazol-2-yl)pyridin-2-yl)piperazine-1-carboxylate (22d)

Intermediate **22c** (500 mg, 1.6 mmol) was added to 50 ml of triethyl orthoformate, and the resulting mixture was heated to 150°C and reacted for 48 h. After the reaction was completed, the crude product was purified by silica gel chromatography (PE/EA= 3/1) to obtain intermediate **22d** (colourless oil, 210 mg, yield: 39%).

### Step 4

2-(6-(piperazin-1-yl)pyridin-3-yl)-1,3,4-oxadiazole (**22e**)

Intermediate **22e** (yellow solid, 82 mg, yield: 86%) was prepared according to the method for synthesizing intermediate **1d**.

### Step 5

### 7-((4-(5-(1,3,4-oxadiazol-2-yl)pyridin-2-yl)piperazin-1-yl)methyl)-3-ethyl-1,5-naphthyridin-2(1H)-one (compound 22)

**Compound 22** (white solid, 42 mg, yield: 81%) was prepared according to the method for synthesizing **compound 1.**

¹H NMR (400 MHz, DMSO) δ 11.86 (s, 1H), 9.24 (s, 1H), 8.70 (d, 1H), 8.41 (d, 1H), 8.05 (dd, 1H), 7.75 (s, 1H), 7.63 (s, 1H), 7.00 (d, 1H), 3.65 (d, 6H), 3.32 (s, 4H), 2.58 - 2.52 (m, 2H), 1.18 (t, 3H)..

LCMS m/s = 418.19 [M+1]

### Example 23

### 7-((4-(6-(difluoromethyl)pyridin-3-yl)piperazin-1-yl)methyl)-3-ethyl-1,5-naphthyridin-2(1H)-one (compound 23)

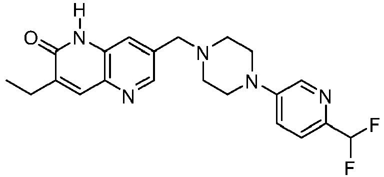

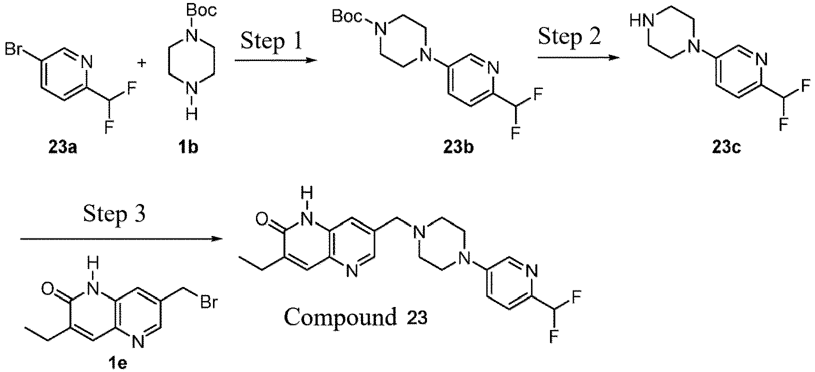

### Step 1

### Tert-butyl 4-(6-(difluoromethyl)pyridin-3-yl)piperazine-1-carboxylate (23b)

5-bromo-2-fluoromethylpyridine (**23a**, 2 g, 9.61 mmol) and tert-butyl piperazine-1-carboxylate (**1b**, 1.79 mg, 9.61 mmol) were dissolved in toluene (30 mL), and then palladium acetate (215 mg, 0.96 mmol) and 2,2'-bis(diphenylphosphino)-1,1'-binaphthyl (598 mg, 0.96 mmol) were added. The reaction flask was subjected to nitrogen replacement and then the mixture was reacted in an oil bath pan at 120°C for 16 h, quenched with water (40 mL) and extracted with ethyl acetate (6 × 30 mL). The organic phases were combined and concentrated under reduced pressure, and the crude product was purified by column chromatography (PE : EA = 5 : 1) to obtain compound **23b** (yellow solid, 1.89 g, yield: 63%).

LCMS m/s = 314.16[M+1].

### Step 2:

### 1-(6-(difluoromethyl)pyridin-3-yl)piperazine (23c)

To compound **23b** (1.89 g, 6.03 mmol) was added hydrogen chloride 1,4-dioxane solution (4 M, 63.3 mL), and the mixture was stirred and reacted at room temperature for 16 h. The reaction liquid was filtered, and the filter cake was collected to obtain compound **23c** (yellow solid, 1.96 g, yield: 94%).

LCMS m/s = 214.11[M+1].

### Step 3:

### 7-((4-(6-(difluoromethyl)pyridin-3-yl)piperazin-1-yl)methyl)-3-ethyl-1,5-naphthyridin-2(1H)-one (compound 23)

Compound **1e** (white solid, 5 g, yield: 76%) was prepared according to the method for synthesizing **intermediate 14** in patent WO 2021013735, LCMS m/s = 267 [M+1].

Compound **23c** (100 mg, 0.46 mmol) and compound **1e** (83 mg, 0.37 mmol) were dissolved in acetonitrile (5 mL), and *N*,*N*-diisopropylethylamine (181 mg, 1.40 mmol) was added. The reaction system was subjected to nitrogen replacement and then the mixture was reacted in an oil bath pan at 70°C for 3 h. The resulting solution was concentrated under reduced pressure to obtain a crude product, which was then purified by column chromatography (MeOH : DCM = 1 : 60 to 1 : 15) to obtain **compound 23** (white solid, 65 mg, yield: 34%).

¹H NMR (400 MHz, DMSO-d6) δ 11.86 (s, 1H), 8.42 - 8.38 (m, 1H), 8.35 (d, J = 2.7 Hz, 1H), 7.75 (s, 1H), 7.62 (s, 1H), 7.49 (d, J = 8.8 Hz, 1H), 7.44 - 7.39 (m, 1H), 6.95 - 6.65 (m, 1H), 3.64 (s, 2H), 3.32 - 3.27 (m, 4H), 2.59 - 2.51 (m, 6H), 1.18 (t, J = 7.4 Hz, 3H).

LCMS m/s = 400.02[M+1].

### Example 24

### 7-((4-(5-(difluoromethyl)pyridin-2-yl)piperazin-1-yl)methyl)-3-ethyl-1,5-naphthyridin-2(1H)-one (compound 24)

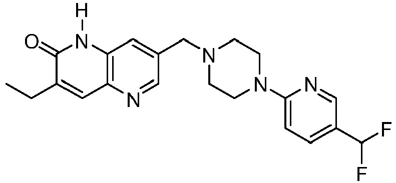

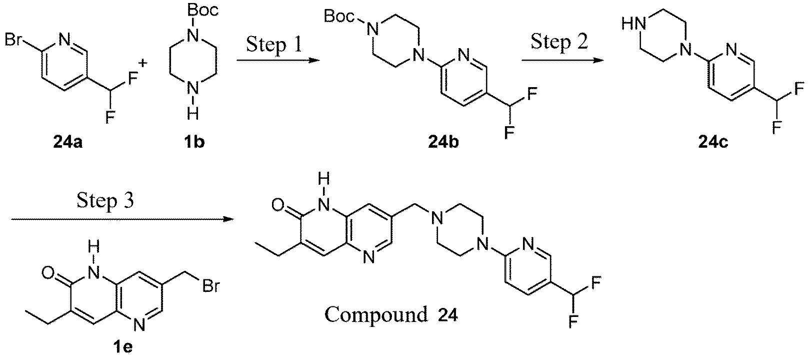

### Step 1

### Tert-butyl 4-(5-(difluoromethyl)pyridin-2-yl)piperazine-1-carboxylate (24b)

Intermediate **24b** (yellow solid, 1.8 g, yield: 85%) was prepared according to the method for synthesizing intermediate **1c.**

LCMS m/s = 314.16[M+1].

### Step 2

### 1-(5-(difluoromethyl)pyridin-2-yl)piperazine (24c)

Intermediate **24d** (yellow solid, 1.1 g, yield: 92%) was prepared according to the method for synthesizing intermediate **1d**.

LCMS m/s = 214.11[M+1].

### Step 3

### 7-((4-(5-(difluoromethyl)pyridin-2-yl)piperazin-1-yl)methyl)-3-ethyl-1,5-naphthyridin-2(1H)-one (compound 24)

**Compound 24** (white solid, 30 mg, yield: 43%) was prepared according to the method for synthesizing **compound 1.**

¹H NMR (400 MHz, DMSO-d6) δ 11.86 (s, 1H), 8.42 - 8.38 (m, 1H), 8.34 (d, J = 2.7 Hz, 1H), 7.75 (s, 1H), 7.62 (s, 1H), 7.48 (d, J = 8.8 Hz, 1H), 7.44 - 7.39 (m, 1H), 6.97 - 6.67 (m, 1H), 3.65 (s, 2H), 3.34 - 3.29 (m, 4H), 2.60 - 2.52 (m, 6H), 1.18 (t, J = 7.4 Hz, 3H).

LCMS m/s = 400.19[M+1].

### Example 25

### 7-((4-(5-(difluoromethoxy)pyridin-2-yl)piperazin-1-yl)methyl)-3-ethyl-l,5-naphthyridin-2(1H)-one (compound 25)

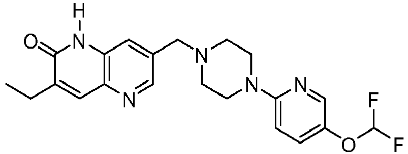

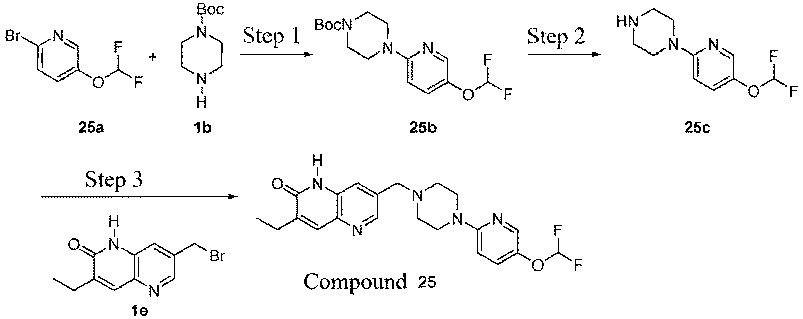

### Step 1

### Tert-butyl 4-(5-(difluoromethoxy)pyridin-2-yl)piperazine-1-carboxylate (25b)

Intermediate **25b** (reddish brown solid, 2.35 g, yield: 79%) was prepared according to the method for synthesizing intermediate **1c.**

LCMS m/s = 330.16[M+1].

### Step 2

### 1-(5-(difluoromethoxy)pyridin-2-yl)piperazine (25c)

Intermediate **25c** (yellow solid, 2.2 g, yield: 95%) was prepared according to the method for synthesizing intermediate **1d**.

LCMS m/s = 230.10[M+1].

### Step 3

### 7-((4-(5-(difluoromethoxy)pyridin-2-yl)piperazin-l-yl)methyl)-3-ethyl-1,5-naphthyridin-2(1H)-one (compound 25)

**Compound 25** (brown solid, 75 mg, yield: 41%) was prepared according to the method for synthesizing **compound 1.**

¹H NMR (400 MHz, DMSO-d6) δ 11.86 (s, 1H), 8.40 (d, J = 1.4 Hz, 1H), 8.01 (d, J = 2.9 Hz, 1H), 7.75 (s, 1H), 7.62 (s, 1H), 7.47 - 7.41 (m, 1H), 7.14 (d, J = 74.3 Hz, 1H), 6.89 - 6.85 (m, 1H), 3.62 (s, 2H), 3.49 - 3.44 (m, 4H), 2.57 - 2.50 (m, 4H), 2.48 - 2.44 (m, 2H), 1.18 (t, J = 7.4 Hz, 3H).

LCMS m/s = 416.18[M+1].

### Example 26

### 6-(4-((7-methyl-6-oxo-5,6-dihydro-1,5-naphthyridin-3-yl)methyl)piperazin-1-yl)nicotinonitrile (compound 26)

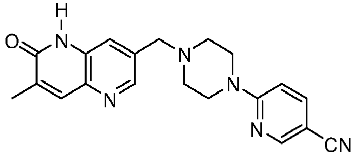

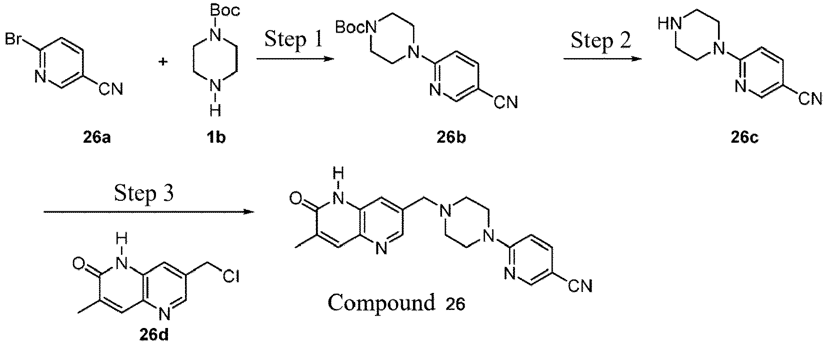

### Step 1

### Tert-butyl 4-(5-cyanopyridin-2-yl)piperazine-1-carboxylate (26b)

Intermediate **26b** (brown solid, 2.47 g, yield: 75%) was prepared according to the method for synthesizing intermediate **1c.**

LCMS m/s = 289.16[M+1].

### Step 2

### 6-(piperazin-1-yl)nicotinonitrile (26c)

Intermediate **26c** (yellow solid, 2.1 g, yield: 92%) was prepared according to the method for synthesizing intermediate **1d**.

LCMS m/s = 189.11[M+1].

### Step 3

### 6-(4-((7-methyl-6-oxo-5,6-dihydro-1,5-naphthyridin-3-yl)methyl)piperazin-1-yl)nicotinonitrile (compound 26)

### Compound 26d was prepared according to the patent WO 2021013735 A1

**Compound 26** (white solid, 88 mg, yield: 33%) was prepared according to the method for synthesizing **compound 1.**

¹H NMR (400 MHz, DMSO-d6) δ 11.87 (s, 1H), 8.47 (d, J = 2.3 Hz, 1H), 8.38 (d, J = 1.6 Hz, 1H), 7.86 - 7.80 (m, 2H), 7.61 (s, 1H), 6.92 (d, J = 9.2 Hz, 1H), 3.69 - 3.64 (m, 4H), 3.62 (s, 2H), 2.49 - 2.45 (m, 4H), 2.13 (s, 3H).

LCMS m/s = 361.17[M+1].

### Example 27

### 2-(4-((7-ethyl-6-oxo-5,6-dihydro-1,5-naphthyridin-3-yl)methyl)piperazin-1-yl)thiazole-5-carbonitrile (compound 27)

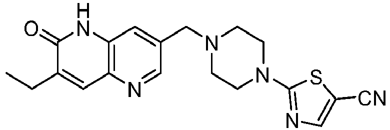

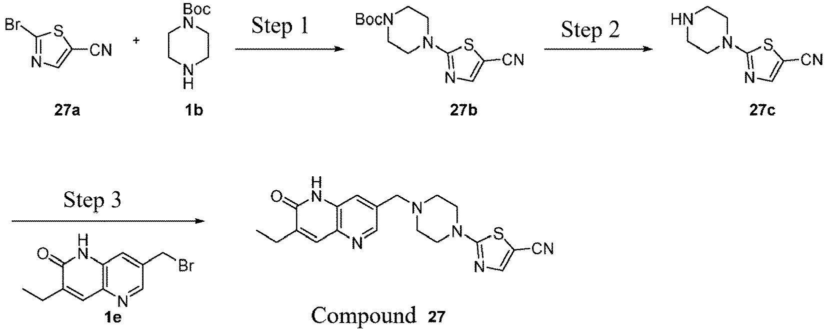

### Step 1

### Tert-butyl 4-(5-cyanothiazol-2-yl)piperazine-1-carboxylate (27b)

2-bromothiazole-5-nitrile (**27a**, 1 g, 5.30 mmol) and tert-butyl piperazine-1-carboxylate (**1b**, 1.08 g, 5.82 mmol) were dissolved in toluene (15 mL), and palladium acetate (119 mg, 0.53 mmol), 2,2'-bis(diphenylphosphino)-1,1'-binaphthyl (330 mg, 0.53 mmol) and caesium carbonate (6.9 g, 21.3 mmol) were added. The reaction flask was subjected to nitrogen replacement and then the mixture was reacted in an oil bath pan at 120°C for 16 h, quenched with water (20 mL) and extracted with ethyl acetate (3 × 30 mL). The organic phases were combined and concentrated under reduced pressure, and the crude product was purified by column chromatography (PE : EA = 5 : 1) to obtain compound **27b** (yellow solid, 1.5 g, yield: 96%).

¹H NMR (400 MHz, DMSO-*d*₆) δ 8.05 (s, 1H), 3.57 - 3.51 (m, 4H), 3.50 - 3.43 (m, 4H), 1.42 (s, 9H).

LCMS m/s = 294.10[M+1].

### Step 2

### 2-(piperazin-1-yl)thiazole-5-carbonitrile (compound 27c)

To compound **27b** (1.5 g, 5 mmol) was added hydrogen chloride 1,4-dioxane solution (4 M, 15 mL), and the mixture was stirred and reacted at room temperature for 2 h. The reaction liquid was filtered, and the filter cake was collected to obtain compound **27c** (yellow solid, 900 mg, yield: 91%).

LCMS m/s = 194.10[M+1].

### Step 3

### 2-(4-((7-ethyl-6-oxo-5,6-dihydro-1,5-naphthyridin-3-yl)methyl)piperazin-1-yl)thiazole-5-carbonitrile (compound 27)

Compound **1e** (white solid, 5 g, yield: 76%) was prepared according to the method for synthesizing **intermediate 14** in patent WO 2021013735, LCMS m/s = 267 [M+1].

Compound **27c** (100 mg, 0.51 mmol) and compound **1e** (123 mg, 0.46 mmol) were dissolved in acetonitrile (5 mL), and *N*,*N*-diisopropylethylamine (297 mg, 2.3 mmol) was added. The reaction system was subjected to nitrogen replacement and then reacted in an oil bath pan at 90°C for 3 h. The resulting solution was concentrated under reduced pressure to obtain a crude product, which was then purified by column chromatography (MeOH : DCM = 1 : 60 to 1 : 15) to obtain **compound 27** (white solid, 100 mg, yield: 57%).

¹H NMR (400 MHz, DMSO-*d*₆) δ 11.88 (s, 1H), 8.39 (d, 1H), 8.03 (s, 1H), 7.75 (s, 1H), 7.60 (d, 1H), 3.65 (s, 2H), 3.60 - 3.50 (m, 4H), 2.54 - 2.52 (m, 6H), 1.17 (t, 3H).

LCMS m/s = 381.20 [M+1].

### Example 28

### 2-(4-((7-ethyl-6-oxo-5,6-dihydro-1,5-naphthyridin-3-yl)methyl)piperazin-1-yl)thiazole-4-carbonitrile (compound 28)

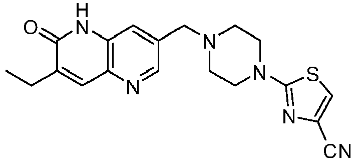

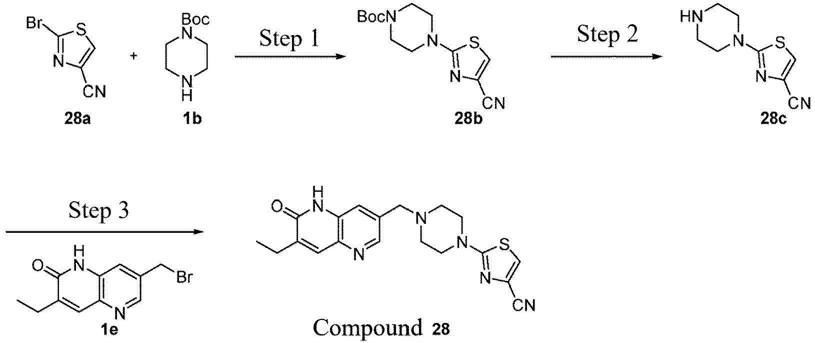

### Step 1

### Tert-butyl 4-(4-cyanothiazol-2-yl)piperazine-1-carboxylate (compound 28b)

2-bromothiazole-4-nitrile (**28a**, 1 g, 5.30 mmol) and tert-butyl piperazine-1-carboxylate (**1b**, 1.08 g, 5.82 mmol) were dissolved in toluene (15 mL), and palladium acetate (119 mg, 0.53 mmol), 2,2'-bis(diphenylphosphino)-1,1'-binaphthyl (330 mg, 0.53 mmol) and caesium carbonate (6.9 g, 21.3 mmol) were added. The reaction flask was subjected to nitrogen replacement and then the mixture was reacted in an oil bath pan at 120°C for 16 h, quenched with water (20 mL) and extracted with ethyl acetate (3 × 30 mL). The organic phases were combined and concentrated under reduced pressure, and the crude product was purified by column chromatography (PE : EA = 5 : 1) to obtain compound **28b** (yellow solid, 100 mg, yield: 6%).

¹H NMR (400 MHz, DMSO-*d*₆) δ 8.00 (s, 1H), 3.44 (s, 8H), 1.42 (s, 10H).

LCMS m/s = 294.10[M+1].

### Step 2

### 2-(piperazin-1-yl)thiazole-4-carbonitrile (compound 28c)

To compound **28b** (100 mg, 0.33 mmol) was added hydrogen chloride 1,4-dioxane solution (4 M, 2 mL), and the mixture was stirred and reacted at room temperature for 2 h. The reaction liquid was filtered, and the filter cake was collected to obtain compound **28c** (yellow solid, 60 mg, yield: 92%).

LCMS m/s = 194.10[M+1].

### Step 3

### 2-(4-((7-ethyl-6-oxo-5,6-dihydro-1,5-naphthyridin-3-yl)methyl)piperazin-1-yl)thiazole-4-carbonitrile 2-(4-((3-ethyl-2-oxo-1,2-dihydroquinolin-7-yl)methyl)piperazin-1-yl)thiazole-4-carbonitrile (compound 28)

Compound **28c** (60 mg, 0.31 mmol) and compound **1e** (91 mg, 0.34 mmol) were dissolved in acetonitrile (5 mL), and *N*,*N*-diisopropylethylamine (219 mg, 1.7 mmol) was added. The reaction system was subjected to nitrogen replacement and then reacted in an oil bath pan at 90°C for 3 h. The resulting solution was concentrated under reduced pressure to obtain a crude product, which was then purified by column chromatography (MeOH : DCM = 1 : 60 to 1 : 15) to obtain **compound 28** (white solid, 50 mg, yield: 38%).

¹H NMR (400 MHz, DMSO-*d*₆) δ 11.87 (s, 1H), 8.39 (d, 1H), 7.98 (s, 1H), 7.74 (s, 1H), 7.60 (d, 1H), 3.64 (s, 2H), 3.55 - 3.39 (m, 4H), 2.54 - 2.52 (m, 6H), 1.17 (t, 3H).

LCMS m/s = 381.20 [M+1].

### Example 29

### 7-((4-(6-(difluoromethoxy)pyridin-3-yl)piperazin-1-yl)methyl)-3-ethyl-1,5-naphthyridin-2(1H)-one (compound 29)

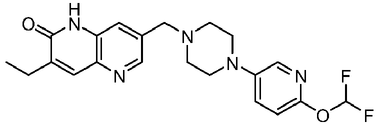

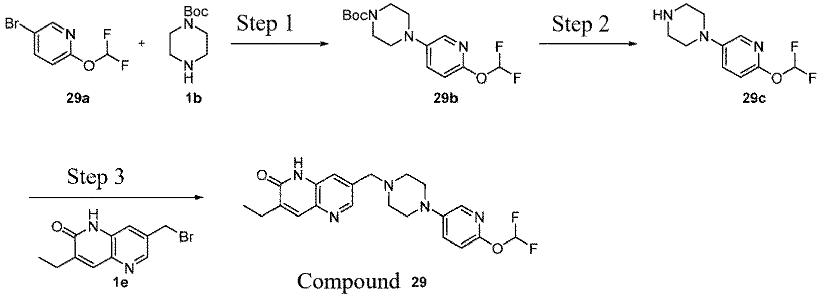

### Step 1

### Tert-butyl 4-(6-(difluoromethoxy)pyridin-3-yl)piperazine-1-carboxylate (compound 29b)

5-bromo-2-(difluoromethoxy)pyridine (**29a**, 500 mg, 2.23 mmol) and tert-butyl piperazine-1-carboxylate (**1b**, 457 mg, 2.45 mmol) were dissolved in toluene (10 mL), and palladium acetate (50 mg, 0.22 mmol), 2,2'-bis(diphenylphosphino)-1,1'-binaphthyl (137 mg, 0.22 mmol) and caesium carbonate (2.9 g, 8.92 mmol) were added. The reaction flask was subjected to nitrogen replacement and then the mixture was reacted in an oil bath pan at 120°C for 16 h, quenched with water (10 mL) and extracted with ethyl acetate (3 × 10 mL). The organic phases were combined and concentrated under reduced pressure, and the crude product was purified by column chromatography (PE : EA = 5 : 1) to obtain compound **29b** (yellow solid, 700 mg, yield: 95%).

¹H NMR (400 MHz, DMSO-*d*₆) δ 7.89 (d, 1H), 7.58 (dd, 1H), 7.56 (t, 1H), 6.98 (d, 1H), 3.47 - 3.44 (m, 4H), 3.14 - 3.03 (m, 4H), 1.42 (s, 9H).

LCMS m/s = 329.20 [M+1].

### Step 2

### 1-(6-(difluoromethoxy)pyridin-3-yl)piperazine (compound 29c)

To compound **29b** (700 mg, 2.13 mmol) was added hydrogen chloride 1,4-dioxane solution (4 M, 4 mL), and the mixture was stirred and reacted at room temperature for 2 h. The reaction liquid was filtered, and the filter cake was collected to obtain compound **29c** (yellow solid, 450 mg, yield: 92%).

LCMS m/s = 229.10 [M+1].

### Step 3

### 7-((4-(6-(difluoromethoxy)pyridin-3-yl)piperazin-1-yl)methyl)-3-ethyl-1,5-naphthyridin-2(1H)-one (compound 29)

Compound **29c** (50 mg, 0.22 mmol) and compound **1e** (52 mg, 0.20 mmol) were dissolved in acetonitrile (5 mL), and N,N-diisopropylethylamine (129 mg, 1.0 mmol) was added. The reaction system was subjected to nitrogen replacement and then reacted in an oil bath pan at 90°C for 3 h. The resulting solution was concentrated under reduced pressure to obtain a crude product, which was then purified by column chromatography (MeOH : DCM = 1 : 60 to 1 : 15) to obtain **compound 29** (white solid, 20 mg, yield: 25%).

¹H NMR (400 MHz, DMSO-*d*₆) δ 11.85 (s, 1H), 8.40 (d, 1H), 7.87 (d, 1H), 7.75 (s, 1H), 7.62 (s, 1H), 7.57 (dd, 1H), 7.54 (t, 1H), 6.96 (d, 1H), 3.64 (s, 2H), 3.22 - 3.07 (m, 4H), 2.56 - 2.53 (m, 6H), 1.18 (t, 3H).

LCMS m/s = 416.20 [M+1].

### Biological Tests

### 1. PARP1 and PARP2 enzyme activity inhibition experiment

In this experiment, the inhibition of a test compound on the enzymatic activities of PARP1 and PARP2 was detected using PARP1 Chemiluminescent assay (BPS, Cat No.: 80551) and PARP2 Chemiluminescent assay (BPS, Cat No.: 80552), respectively. The specific experimental method was as follows: a 96-well plate was coated with a histone mixture overnight, then a blocking buffer was added, and the plate was incubated at room temperature for 90 min. Subsequently, a test compound, a PARP enzyme and a biotinylated substrate were added, and the mixture was incubated at room temperature for 1 h. After the incubation, streptavidin-labelled HRP was added and the resulting mixture was incubated at room temperature for 30 min. Finally, a mixed liquid of ELISA ECL substrates A/B was added, and bioluminescence was immediately detected using a microplate reader. The IC₅₀ value was calculated using GraphPad Prism 8 software.

The results showed that the compound of the present invention had significant biological inhibitory activity on PARP1 and exhibited good selectivity for PARP1 over PARP2.

### 2. PARP1 and PARP2 trapping experiments

### 2.1 PARP1 trapping experiment

PARP1 (BPS, Cat No.: 80501) and Mab anti GST-Tb cryptate (cisbio, Cat No.: 61GSTTL) were first added to a 384-well plate, DSB DNA probe-1 (Generay) and a test compound were then added, and the resulting mixture was incubated at room temperature for 45 min. Subsequently, NAD (Sigma, Cat No.: 10127965001) was added, and the resulting mixture was incubated at room temperature for 10 min. TR-FRET signals were detected using Envision 2105 (PerkinElmer). The IC₅₀ value was calculated using GraphPad Prism 8 software. The results are as shown in Table 1.

### 2.2 PARP2 trapping experiment

PARP2 (BPS, Cat No.: 80502) and Mab anti GST-Tb cryptate (cisbio, Cat No.: 61GSTTL) were first added to a 384-well plate, PARP2 probe2 (Generay) and a test compound were then added, and the resulting mixture was incubated at room temperature for 45 min. Subsequently, NAD (Sigma, Cat No.: 10127965001) was added, and the resulting mixture was incubated at room temperature for 10 min. Finally, TR-FRET signals were detected using Envision 2105 (PerkinElmer). The IC₅₀ value was calculated using GraphPad Prism 8 software. The results are as shown in Table 1.

**Table 1. Results of PARP1 and PARP2 trapping experiments**

| Compounds | PARP1 trapping IC₅₀ (nM) | PARP2 trapping IC₅₀ (nM) | PARP2/PARP1 |
|---|---|---|---|
| Comparative example 1 | 1.9 | 1158 | 619 |
| 1 | 1.0 | 2558 | 2479 |
| 3 | 1.2 | 3052 | 2554 |
| 4 | 1.1 | 2878 | 2586 |
| 13 | 0.8 | 682 | 801 |
| 14 | 0.8 | 1676 | 2028 |

| | | | |
|---|---|---|---|
| Notes: Comparative example 1 is compound 25 from J. Med. Chem (2021), 64(19), 14498-14512, and was obtained according to the method for preparing compound 25. | | | |

The results showed that the compounds of the present invention had significant inhibitory activity on PARP1 trapping and exhibited good selectivity for PARP1 trapping over PARP2 trapping.

### 3. Cell proliferation inhibition experiment

### 3.1 DLD-1 BRCA2^{-/-} cell proliferation inhibition experiment

Epithelial cell line DLD-1 BRCA2^{-/-} from human colorectal adenocarcinoma (Horizon Discovery Ltd., Cat No.: HD105-007) was cultured in an RPMI-1640 medium containing 10% FBS, 1% penicillin-streptomycin solution, 1% L-G and 0.1 mg/mL hygromycin at 37°C and 5% CO₂. By means of cell counting, 1000 DLD-1 BRCA2^{-/-} cells were seeded into each well of a 96-well plate and cultured overnight. Test compounds (2-fold gradient dilution, starting at the highest dose: 10 µM, a total of 9 gradient concentrations) were added the next day. Moreover, blank control wells containing DMSO at the same concentration were set. The cells were cultured at 37°C and 5% CO₂ for 7 d, CellTiter-Glo^{®} Reagent was then added, and the chemiluminescence values were determined using a microplate reader. The IC₅₀ value was calculated using GraphPad Prism 8 software. The results are as shown in Table 2.

**Table 2. DLD-1 BRCA2-/- cell proliferation inhibition experiment of the compounds of the present invention**

| **Compounds** | **IC₅₀ (nM)** |
|---|---|
| **1** | 1.7 |
| **3** | 3.3 |
| **4** | 5.4 |

The results showed that the compounds of the present invention had significant inhibitory effects on the proliferation of DLD1 BRCA2^{-/-} cells.

### 3.2 MDA-MB-436 cell proliferation inhibition experiment

Human breast cancer cell line MDA-MB-436 (supplier: ATCC, Cat#HTB-130) was cultured in an L15 medium containing 10% FBS and 1% penicillin-streptomycin solution at 37°C and 5% CO₂. By means of cell counting, 400 MDA-MB-436 cells were seeded into each well of a 384-well plate and cultured overnight. Test compounds (3-fold gradient dilution, starting at the highest dose: 10 µM, a total of 10 gradient concentrations) were added the next day. Moreover, blank control wells containing 0.1% DMSO were set. The cells were cultured at 37°C and 5% CO₂ for 7 d, a Celltiter Glo assay kit detection solution was then added, and the chemiluminescence values were determined using a microplate reader. The IC₅₀ value was calculated using GraphPad Prism 8 software. The results are as shown in Table 3.

**Table 3. MDA-MB-436 cell proliferation inhibition experiment of the compounds of the present invention**

| **Compound No.** | IC₅₀ (nM) |
|---|---|
| **Comparative example 2** | 29.2 |
| **1** | 12.2 |
| **3** | 11.1 |
| **10** | 12.1 |
| **14** | 1.9 |
| **21** | 27.8 |
| **24** | 8.3 |

| | |
|---|---|
| Notes: **Comparative example 2** is compound 17 from the patent WO 2009053373, and was obtained according to the method for preparing compound 17. | |

The results showed that the compounds of the present invention had significant inhibitory effects on the proliferation of MDA-MB-436 cells.

### 4. Bidirectional permeability evaluation with MDCK-MDR1 cell model

In this experiment, monolayer MDCK-MDR1 cells were used and incubated in a 96-well Transwell plate. A transport buffer containing 1 µM test compound was added to an appropriate administration end well on the apical or basal side, and a DMSO-containing transport buffer was added to an appropriate receiving end well. After incubation at 37°C for 2 h, the cell plate was removed, the samples (50 µL each) were collected from the top and bottom ends and transferred to a new 96-well plate, and the protein was precipitated by subsequently adding acetonitrile. The samples were analysed using LC-MS/MS and the concentration of the test compound was determined. The concentration data were used to calculate the apparent permeability coefficients of the transport from the apical side to the basal side and from the basal side to the apical side of the monolayer cells so as to calculate the efflux rate. The integrity of the monolayer cells was assessed by the leakage of Lucifer Yellow after 2 h of incubation. The penetration results of the test compound in monolayer MDCK-MDR1 cells were as shown in Table 4.

**Table 4. Penetration results of the compound of the present invention in monolayer MDCK-MDR1 cells.**

| Compound No. | MDCK-MDRII Papp (A-B) (10-6, cm/s) | MDCK-MDRII Papp (B-A) (10-6, cm/s) | Efflux Ratio |
|---|---|---|---|
| **Comparative example 1** | 3.38 | 43.78 | 13.06 |
| **Compound 14** | 16.43 | 37.33 | 2.3 |

| | | | |
|---|---|---|---|
| Notes: Comparative example 1 is **compound 25** from J. Med. Chem (2021), 64(19), 14498-14512, and was obtained according to the method for preparing compound 25. | | | |

The results showed that: compared with the comparative example, the compound of the present invention had higher cell membrane permeability and lower efflux rate.

### 5. Pharmacokinetic test in mice

An appropriate amount of the test compound was precisely weighed and formulated into a 0.1 mg/mL solution with 30% DMSO and 30% HP-*β*-CD for intravenous injection, and the test compound was formulated into a 0.1 mg/mL solution with 0.5% MC for intragastric administration. Healthy adult male ICR mice were fasted overnight and then given the test compound via intravenous injection (0.5 mg/kg) and intragastric administration (1 mg/kg). Blood (anticoagulated with heparin sodium) was collected from the forelimb vein at different time points (0.083 h, 0.25 h, 0.5 h, 1 h, 2 h, 4 h, 8 h and 24 h) after administration. The blood samples were separated by centrifugation at 6800 g at 4°C for 10 min to obtain plasma, and all the samples were stored at - 80°C for later testing. The concentration of the prototype drug in plasma was determined by an LC-MS/MS method, and the main pharmacokinetic parameters were calculated using Phoenix WinNonlin7.0. The results are as shown in Table 5.

**Table 5. Pharmacokinetic properties of the compound of the present invention in ICR mice**

| Compound No. | Oral administration (1 mg/kg) | | |
|---|---|---|---|
| | Cₘₐₓ (µg/mL) | AUC₀₋ₜ (µg*h/mL) | T_{1/2} (h) |
| Comparative example 1 | 5.41 | 47.56 | 6.25 |
| Compound 14 | 13.2 | 183.33 | 20.36 |

| | | | |
|---|---|---|---|
| Notes: Comparative example 1 is **compound 25** from J. Med. Chem (2021), 64(19), 14498-14512, and was obtained according to the method for preparing compound 25. | | | |

The results showed that: the compound of the present application exhibited significantly better pharmacokinetic characteristics in mice compared with the comparative example.

### 6. Compound distribution detection in rat brain tissue

An appropriate amount of the test compound was precisely weighed and formulated into a 0.2 mg/mL solution with 0.5% MC. Healthy adult male SD rats were fasted overnight and then given the test compound (1 mg/kg) or a blank solvent via intragastric administration. Blood was collected from the orbital venous plexus (anticoagulated with EDTA-K₂) at different time points after administration. The animals were subsequently sacrificed, and brain tissue samples were collected. The blood samples were separated by centrifugation at 6000 g at 4°C for 5 min to obtain plasma, and all the samples were stored at -80°C for later testing. The drug concentration of the compound in the brain tissue was determined by an LC-MS/MS method, and the brain tissue samples were homogenized prior to determination. The main pharmacokinetic parameters were calculated using a Winnolin 8.3 noncompartmental model.

The results showed that: the compound of the present application exhibited a good blood-brain barrier penetration ability.

### 7. Pharmacodynamic test on DLD-1 BRCA2-/- transplanted tumours

Epithelial cells DLD-1 BRCA2-/- from human colorectal adenocarcinoma were cultured in an RPMI-1640 medium containing 10% FBS and 1% penicillin-streptomycin solution. When the cell confluence reached 80%-90%, the cells were digested, centrifuged and counted. The matrigel and the cell suspension were mixed uniformly at the ratio of 1 : 1 and the resulting mixture was inoculated subcutaneously into the right flank of BALB/c nude mice at an inoculation amount of 5 × 10⁶/mouse and an inoculation volume of 0.2 mL. When the tumours grew to about 160 mm³, the mice were divided into 6 groups (8 mice/group) in total according to the tumour size (in a zigzag pattern): vehicle, comparative example 1 (0.1 mpk, 1 mpk and 10 mpk) and compound 14 (1 mpk and 10 mpk). The test compound was formulated with 0.5% MC and was intragastrically administered at an administration volume of 10 mL/kg, once a day for a total of 28 days. During the administration, the tumour volume and body weight of the mice were measured twice a week, and tumour growth curves and mouse body weight change curves were plotted. At the last administration, blood samples were collected from the orbital venous plexus of mice at different time points (0 h, 0.5 h, 1 h, 2 h, 4 h, 6 h, 8 h, 12 h and 24 h) and centrifuged to obtain the plasma. The drug concentration was then determined. In addition, during the last administration, tumours were excised from mice 1 h, 6 h and 24 h after administration and ground, and the drug concentration in the tumours was then determined.

The results showed that: compared with the comparative example, the compound of the present application had a higher concentration in the tumour tissue, and the compound of the present application exhibited significantly better efficacy in the transplanted tumour model compared with the comparative example.

Specific embodiments have been described in detail in the specification of the present invention. A person skilled in the art should recognize that the above-mentioned embodiments are illustrative and cannot be understood as limitations to the present invention. A person skilled in the art can make several improvements and modifications to the present invention without departing from the principle of the present invention, and the technical solutions obtained based on these improvements and modifications also fall within the scope of protection of the claims of the present invention.

## Claims

1. A compound represented by general formula (I-A), or a stereoisomer, a pharmaceutically acceptable salt or a deuterated compound thereof: 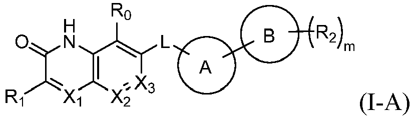 **characterised in that**:
R₁ is selected from C₁₋₆ alkyl, C₂₋₆ alkenyl, C₂₋₆ alkynyl, C₃₋₈ cycloalkyl or C₃₋₈ heterocycloalkyl, wherein the C₃₋₈ heterocycloalkyl may contain 1 to 4 heteroatoms selected from N, O or S;
R₀ is selected from H, halogen or C₁₋₆ alkyl, wherein the C₁₋₆ alkyl is optionally further substituted with one or more substituents selected from halogen;
X₁, X₂ and X₃ are each independently selected from N or CRx, and at least one of X₁, X₂ and X₃ is selected from N;
Rx is selected from H, halogen, hydroxyl, cyano, C₁₋₆ alkyl, C₁₋₆ alkoxy or C₃₋₈ cycloalkyl;
L is selected from CH₂;
A is a 4- to 12-membered heterocycle selected from a 4- to 12-membered monocyclic ring, a 5- to 12-membered spiro ring, a 4- to 12-membered fused ring, or a 4- to 12-membered bridged ring, wherein the 4- to 12-membered heterocycle may contain 1 to 4 heteroatoms selected from N, O or S;
structural fragment 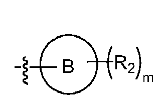 is selected from 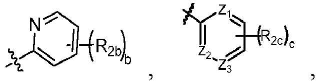 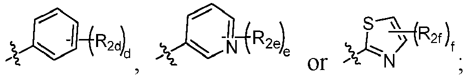
R_{2b} may be the same or different;
R_{2c} may be the same or different;
R_{2d} may be the same or different;
R₂ₑ may be the same or different;
R_{2f} may be the same or different;
R_{2b}, R_{2c} and R_{2f} are each independently selected from CN, halogen, OR₂ₐ, C₁₋₆ alkyl or a 4-to 12-membered heterocycle, wherein the C₁₋₆ alkyl and 4- to 12-membered heterocycle are optionally further substituted with one or more substituents selected from halogen, OH and C₁₋₃ alkyl, and the 4- to 12-membered heterocycle may contain 1 to 4 heteroatoms selected from N, O or S;
R_{2d} and R₂ₑ are each independently selected from CN, halogen, OR₂ₐ and C₁₋₆ alkyl, wherein the C₁₋₆ alkyl is optionally further substituted with one or more substituents selected from halogen or OH;
R₂ₐ is selected from H, C₁₋₆ alkyl, (CH₂)ₙC₃₋₈ cycloalkyl or (CH₂)ₙC₃₋₈ heterocycloalkyl, wherein the C₃₋₈ heterocycloalkyl may contain 1 to 4 heteroatoms selected from N, O or S, and the C₁₋₆ alkyl is optionally further substituted with one or more substituents selected from halogen;
Z₁, Z₂ and Z₃ are each independently selected from N or C, and at least two of Z₁, Z₂ and Z₃ are selected from N;
n is selected from 0, 1, 2 or 3;
b is selected from 1, 2 or 3;
c is selected from 1, 2 or 3;
d is selected from 2 or 3;
e is selected from 1, 2 or 3;
f is selected from 1 or 2,
provided that:
the compound represented by general formula (I-A) is not: 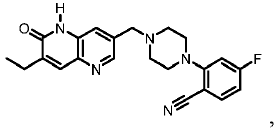 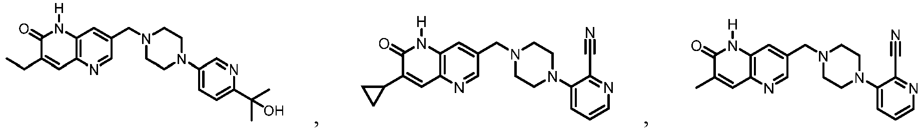 or

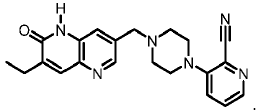

2. The compound, or the stereoisomer, the pharmaceutically acceptable salt or the deuterated compound thereof according to claim 1, **characterised in that**:
structure unit 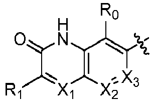 is selected from 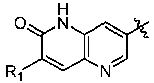 or 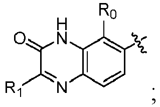
R₁ is selected from C₁₋₆ alkyl, C₂₋₆ alkenyl or C₃₋₈ cycloalkyl;
R₀ is selected from halogen;
A is selected from 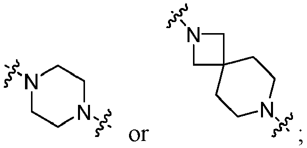
R_{2b} may be the same or different, and each R_{2b} is independently selected from CN, halogen, C₁₋₃ alkoxy, C₁₋₃ alkyl or a 4- to 12-membered heterocycle, wherein the C₁₋₃ alkyl, C₁₋₃ alkoxy and 4- to 12-membered heterocycle are optionally further substituted with one or more substituents selected from halogen, OH and C₁₋₃ alkyl, and the 4- to 12-membered heterocycle may contain 1 to 4 heteroatoms selected from N, O or S;
R_{2c} is CN;
R_{2d} is CN or halogen;
R_{2f} is CN;
R₂ₑ may be the same or different, and each R₂ₑ is independently selected from CN, halogen, OR₂ₐ and C₁₋₆ alkyl, wherein the C₁₋₆ alkyl is optionally further substituted with one or more substituents selected from halogen or OH;
R₂ₐ is selected from C₁₋₃ alkyl or 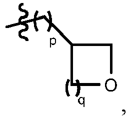 wherein the C₁₋₃ alkyl is optionally further substituted with one or more substituents selected from halogen;
p is selected from 0 or 1;
q is selected from 1 or 2.

3. The compound, or the stereoisomer, the pharmaceutically acceptable salt or the deuterated compound thereof according to claim 2, **characterised in that**:
structure unit 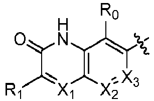 is selected from 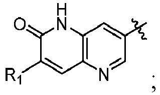
R₁ is selected from C₁₋₆ alkyl or C₃₋₈ cycloalkyl;
A is selected from 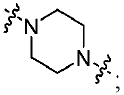
R₂ₑ may be the same or different, and each R₂ₑ is independently selected from CN, OR₂ₐ or C₁₋₃ alkyl, wherein the C₁₋₆ alkyl is optionally further substituted with one or more substituents selected from halogen;
R₂ₐ is selected from C₁₋₃ alkyl or 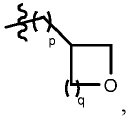 wherein the C₁₋₃ alkyl is optionally further substituted with one or more substituents selected from halogen.

4. The compound, or the stereoisomer, the pharmaceutically acceptable salt or the deuterated compound thereof according to claim 3, **characterised in that**:
R_{2b} is selected from CN, halogen, C₁₋₃ alkyl or a 5-membered heterocycle, wherein the C₁₋₃ alkyl and 5-membered heterocycle are optionally further substituted with one or more substituents selected from halogen and C₁₋₃ alkyl, and the 5-membered heterocycle may contain 1 to 4 heteroatoms selected from N, O or S;
R₂ₑ is selected from CN.

5. The compound, or the stereoisomer, the pharmaceutically acceptable salt or the deuterated compound thereof according to claim 4, **characterised in that**: R_{2b} is selected from CN.

6. A compound represented by general formula (I) or a stereoisomer thereof: 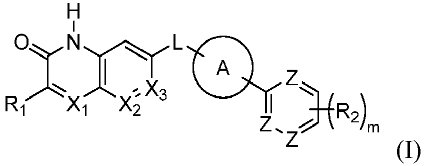 **characterised in that**:
R₁ is selected from C₁₋₆ alkyl, C₂₋₆ alkenyl, C₂₋₆ alkynyl, C₃₋₈ cycloalkyl or C₃₋₈ heterocycloalkyl, wherein the C₃₋₈ heterocycloalkyl may contain 1 to 4 heteroatoms selected from N, O or S;
X₁, X₂ and X₃ are each independently selected from N or CRx;
Rx is selected from H, halogen, hydroxyl, cyano, C₁₋₆ alkyl, C₁₋₆ alkoxy or C₃₋₈ cycloalkyl;
L is selected from CH₂;
A is a 4- to 12-membered heterocycle selected from a 4- to 12-membered monocyclic ring, a 5- to 12-membered spiro ring, a 4- to 12-membered fused ring, or a 4- to 12-membered bridged ring, wherein the 4- to 12-membered heterocycle may contain 1 to 4 heteroatoms selected from N, O or S;
R₂ may be the same or different, and each R₂ is independently selected from CN, halogen, OR₂ₐ or C₁₋₆ alkyl;
R₂ₐ is selected from H, C₁₋₆ alkyl, (CH₂)ₙC₃₋₈ cycloalkyl or (CH₂)ₙC₃₋₈ heterocycloalkyl, wherein the C₃₋₈ heterocycloalkyl may contain 1 to 4 heteroatoms selected from N, O or S, and the C₁₋₆ alkyl is optionally further substituted with one or more substituents selected from halogen;
Z may be the same or different, and each Z is independently selected from CH or N;
m is selected from 1, 2 or 3;
n is selected from 0, 1, 2 or 3,
provided that:
the compound represented by general formula (I) is not

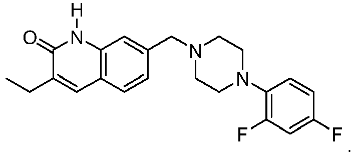

7. A compound represented by general formula (II) or a stereoisomer thereof: 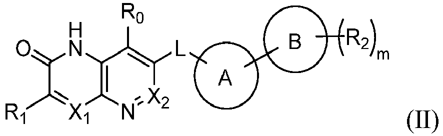 **characterised in that**:
R₁ is selected from C₁₋₆ alkyl, C₂₋₆ alkenyl, C₂₋₆ alkynyl, C₃₋₈ cycloalkyl or C₃₋₈ heterocycloalkyl, wherein the C₃₋₈ heterocycloalkyl may contain 1 to 4 heteroatoms selected from N, O or S;
R₀ is selected from H, halogen or C₁₋₆ alkyl, wherein the C₁₋₆ alkyl is optionally further substituted with one or more substituents selected from halogen or C₁₋₆ alkyl;
X₁ and X₂ are each independently selected from Nor CRx;
Rx is selected from H, halogen, hydroxyl, cyano, C₁₋₆ alkyl, C₁₋₆ alkoxy or C₃₋₈ cycloalkyl;
L is selected from CH₂;
A is a 4- to 12-membered heterocycle selected from a 4- to 12-membered monocyclic ring, a 5- to 12-membered spiro ring, a 4- to 12-membered fused ring, or a 4- to 12-membered bridged ring, wherein the 4- to 12-membered heterocycle may contain 1 to 4 heteroatoms selected from N, OorS;
B is selected from 6-membered aryl or heteroaryl, wherein the heteroaryl may contain 1 to 4 heteroatoms selected from N;
R₂ may be the same or different, and each R₂ is independently selected from CN, halogen, OR₂ₐ or C₁₋₆ alkyl;
R₂ₐ is selected from H, C₁₋₆ alkyl, (CH₂)ₙC₃₋₈ cycloalkyl or (CH₂)ₙC₃₋₈ heterocycloalkyl, wherein the C₃₋₈ heterocycloalkyl may contain 1 to 4 heteroatoms selected from N, O or S, and the C₁₋₆ alkyl is optionally further substituted with one or more substituents selected from halogen;
Z may be the same or different, and each Z is independently selected from CH or N;
m is selected from 1, 2 or 3;
n is selected from 0, 1, 2 or 3.

8. The compound or the stereoisomer thereof according to any one of claims 1 to 7, **characterised in that** the compound is selected from: 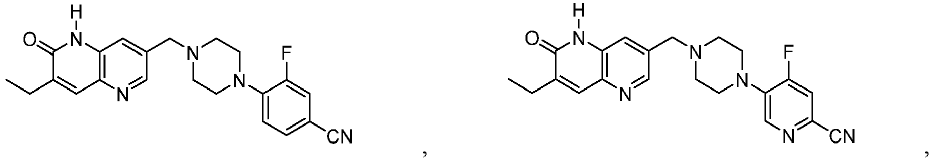 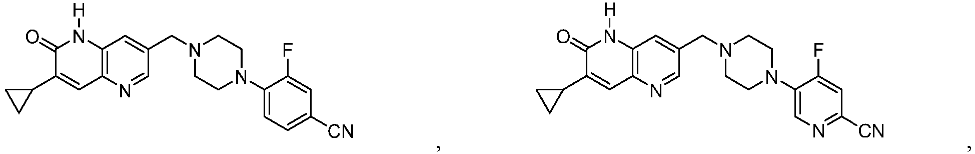 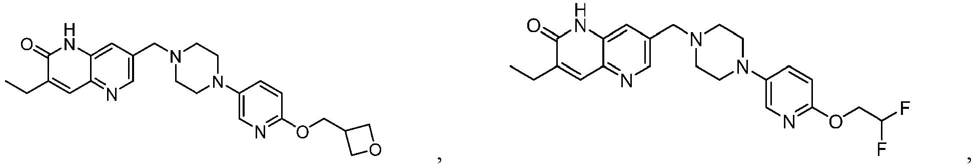 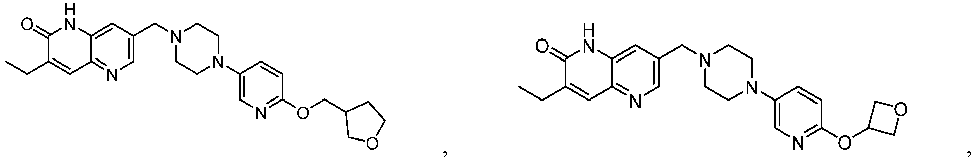 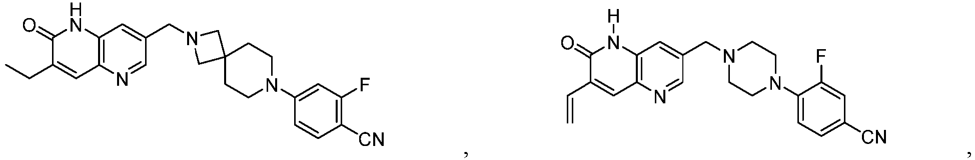 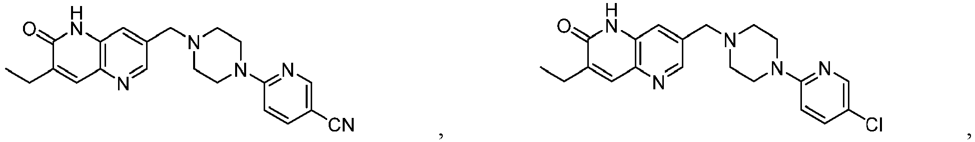 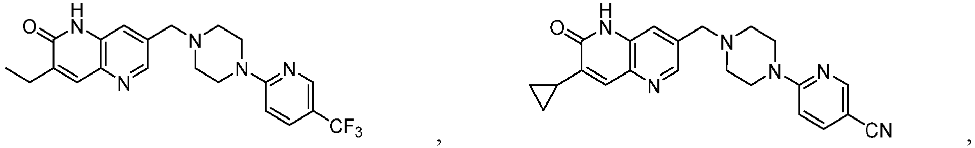 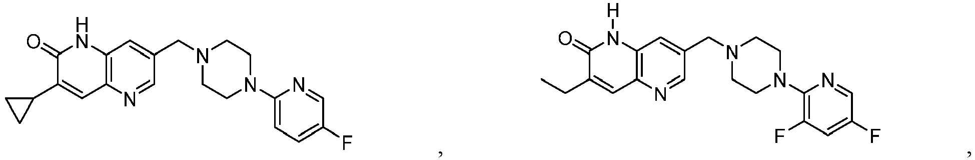 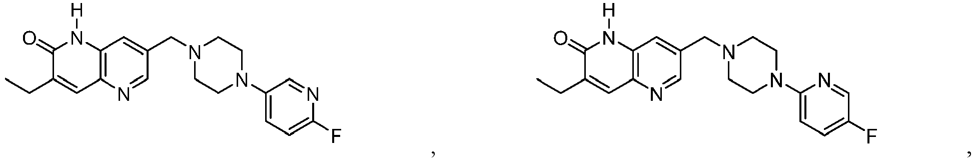 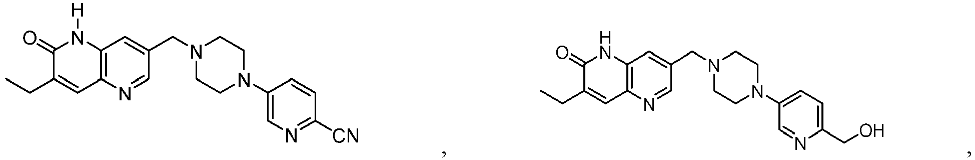 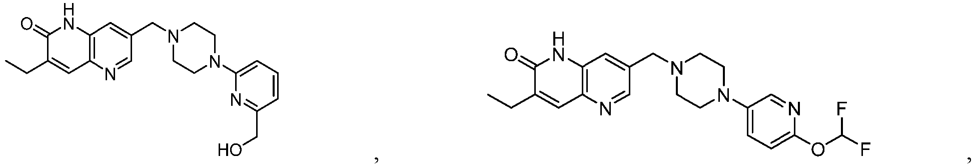 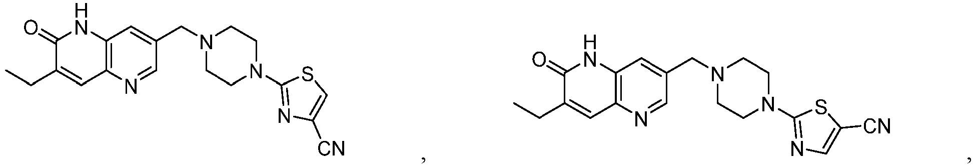 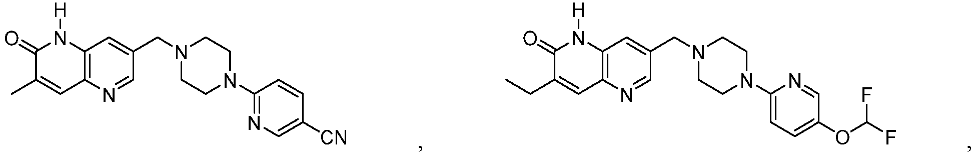 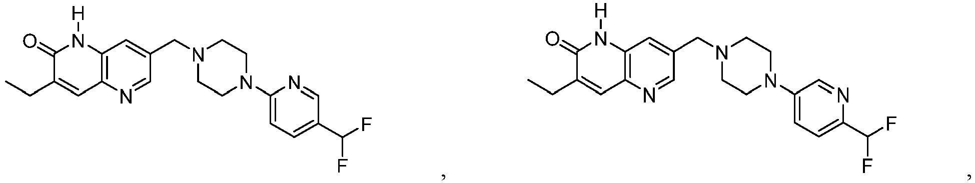 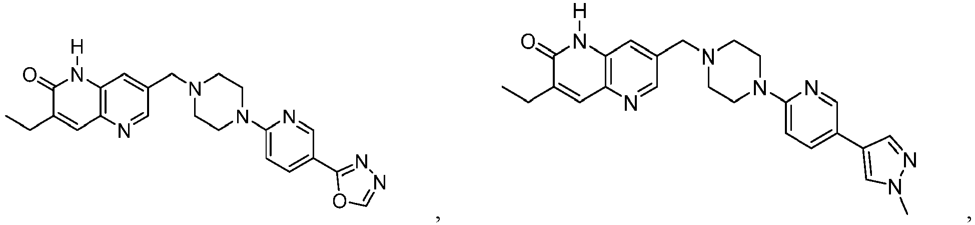 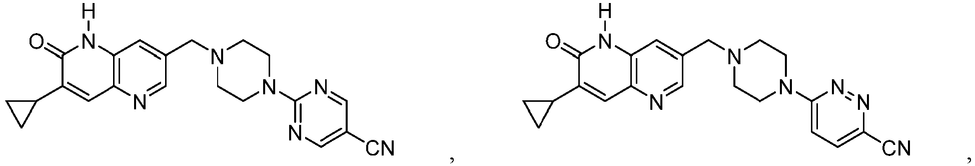 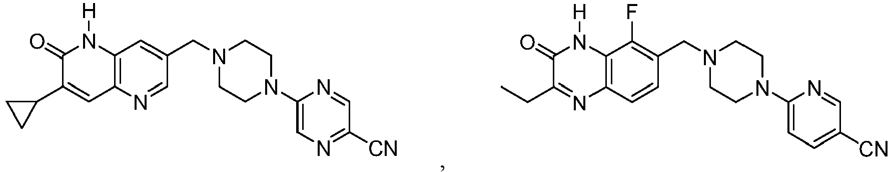 and

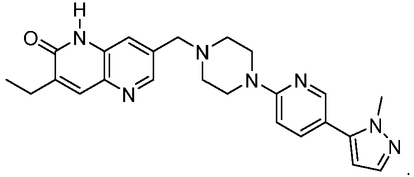

9. A pharmaceutical composition, **characterised in that** it comprises:
(1) the compound or the stereoisomer thereof according to any one of claims 1 to 8;
(2) optionally one or more additional active ingredients; and
(3) a pharmaceutically acceptable carrier and/or excipient.

10. Use of the pharmaceutical composition according to claim 9 or the compound or the stereoisomer thereof according to any one of claims 1 to 8 in the preparation of anti-tumour drugs.
